Europäisches Patentamt

European Patent Office

Office européen des brevets

(11) Publication number: **0 297 970 B1**

(12) **EUROPEAN PATENT SPECIFICATION**

(45) Date of publication of patent specification: **12.10.94** (51) Int. Cl.⁵: **A61M 1/36**, F28D 7/00

(21) Application number: **88401641.1**

(22) Date of filing: **27.06.88**

Divisional application 92116352.3 filed on 27/06/88.

(54) **Heat exchanger for medical treatment.**

(30) Priority: **28.06.87 JP 160094/87**
**27.07.87 JP 187040/87**
**31.07.87 JP 116765/87 U**
**31.07.87 JP 116766/87 U**
**16.09.87 JP 140073/87 U**

(43) Date of publication of application:
**04.01.89 Bulletin 89/01**

(45) Publication of the grant of the patent:
**12.10.94 Bulletin 94/41**

(84) Designated Contracting States:
**BE FR GB IT SE**

(56) References cited:
**DE-A- 1 501 620          FR-A- 2 584 609**
**GB-A- 1 570 056          GB-A- 2 027 864**
**GB-A- 2 157 820          US-A- 4 722 829**

(73) Proprietor: **TERUMO KABUSHIKI KAISHA**
**No. 44-1, Hatagaya 2-chome,**
**Shibuya-ku**
**Tokyo 151 (JP)**

(72) Inventor: **Oshiyama Hiroki**
**c/o Terumo Kabushiki Kaisha, 2656-1 Oh-buchi**
**Fuji-shi, Shizuoka-ken (JP)**
Inventor: **Nogawa Atsuhiko**
**c/o Terumo Kabushiki Kaisha, 2656-1 Oh-buchi**
**Fuji-shi, Shizuoka-ken (JP)**

(74) Representative: **Dossmann, Gérard et al**
**Bureau D.A. Casalonga-Josse**
**Morassistrasse 8**
**D-80469 München (DE)**

EP 0 297 970 B1

**Description**

BACKGROUND OF THE INVENTION

Field of the Invention:

This invention relates to a heat exchanger for medical treatment. More particularly, it relates to a heat exchanger for medical treatment to be used in the extracorporeal circulation of blood for the purpose of maintaining the temperature of the blood at a desired level during the course of the circulation.

Description of the Prior Art:

The extracorporeal blood circulation is generally employed as an auxiliary measure for surgery of the heart, particularly for cardiotomy. In the extracorporeal blood circulation as an auxiliary measure for cardiotomy, the blood drawn out of the patient's body is forwarded to an oxygenator, there to be oxygenated, and then returned in an oxygen-saturated state back to the patient's body. In the surgical operation performed on a complicated infantile cardiac deformation or on an adult aortic aneurysm, for example, the extremely low body temperature extracorporeal circulation method or the medial body temperature extracorporeal circulation method is employed. This blood circulation is effected by cooling the blood drawn, out of the patient's body. In the extracorporeal blood circulation of the nature under discussion, the blood drawn out of the patient's body must be kept at a prescribed temperature or cooled or heated. For this purpose, it has been customary to use a heat exchanger as inserted in the circuit for the extracorporeal blood circulation.

The heat exchangers developed to date for use in the extracorporeal blood circulation are widely varied in type. For example FR-A-2 584 609 (HOSPAL) discloses a flat type heat-exchanger for blood using a linear array of flat tubes arranged within a flat housing, the inlet tube arrives within a chamber having a decreasing cross section which permits a substantially uniform distribution of the blood within gaps defined between the flat tubes.

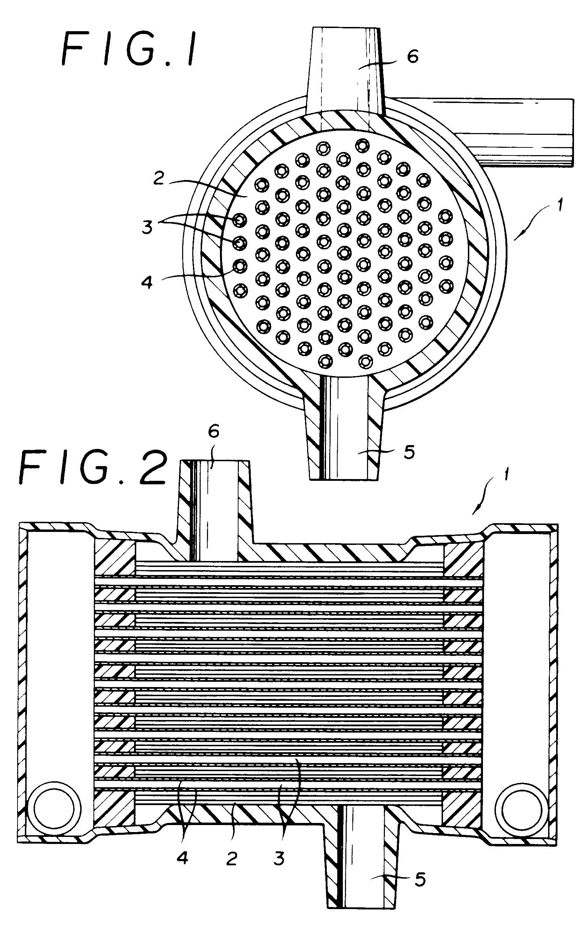

Another example is a shell-and-tube exchanger 1 which, as illustrated in Figs. 1 and 2, comprises a first fluid passing space 2 and a multiplicity of heat-exchanging tubes 4 disposed inside the first fluid passing space 2 in the longitudinal direction of the first fluid passing space 2 and provided with an inner space 3 destined to form a second fluid passing space watertightly separated from the first fluid passing space 2 this heat-exchanger being a highly hopeful heat exchanger for medical treatment which effects very efficient exchange of heat and enjoys exceptional compactness of design. When the first fluid passing space 2 is formed in a cylindrical shape, a first fluid inlet tube 5 for introducing a first fluid into the first fluid passing space 2 and a first fluid outlet tube 6 for discharging the first fluid from the first fluid passing space 2 are adapted, as illustrated in Figs. 1 and 2, so as to be extended inwardly from outside substantially along the straight line passing the central part of a cross section perpendicular to the axis of the first fluid passing space 2 and consequently allowed to communicated with the first fluid passing space 2.

When the shell-and-tube exchanger 1 constructed as described above is used in effecting exchange of heat between the blood and the heat-exchanging medium by passing the heat-exchanging medium through the first fluid passing space 2 and the blood through the second fluid passing space, i.e., the inner spaces 3 of the heat-exchanging tubes 4, the exchange of heat can be accomplished substantially uniformly on the whole blood because the blood is distributed comparatively uniformly and allowed to keep a relatively constant contact with respect to the heat-exchanging medium. When the pressure loss during the introduction of the blood is large and the extracorporeal blood circulation lasts for a long time, there is a fair possibility that the blood will be coagulated inside the inner spaces 3 of the heat-exchanging tubes 4 and will consequently clog or constrict the inner spaces 3. Conversely, when the exchange of heat is carried out by passing the blood through the first fluid passing space 2 and the heat-exchanging medium through the second fluid passing space or the inner spaces 3 of the heat-exchanging tubes 4, the aforementioned possibility of the blood conduits being clogged or constricted is substantially precluded and the pressure loss due to the introduction of the blood is repressed to a comparatively large extent. Since the first fluid inlet tube 5 and the first fluid outlet tube 6 are adapted, as described above, so as to be extended inwardly from outside substantially along the straight line passing the central part of a cross section perpendicular to the axis of the first fluid passing space 2 and consequently allowed to communicate with the first fluid passing space 2, the blood mainly advances toward the central part of the first fluid passing space 2 and consequently the flow of the blood inside the first fluid passing space 2 is not uniformized but is varied locally. In the region of relatively high blood flow, exchange of heat is effected to an unduly small extent

2

because the blood does not sufficiently contact the heat-exchanging tubes 4 now passing the heat-exchanging medium inside the inner spaces 3 thereof. By contrast, in the region of relatively low blood flow, the exchange of heat is effected to an unduly large extent because the blood contacts the heat-exchanging tubes 4 more than is normally required. Where the heat-exchanging tubes 4 are distributed throughout the whole blood passing space 2 as illustrated in Figs. 1 and 2, the unfavorable situation mentioned above grows in conspicuity because the blood introduced through the blood inlet tube 5, on entering the blood passing space 2, comes into direct contact with the heat-exchanging tubes 4 and, consequently, the blood flow is not given a chance enough to be uniformly distributed throughout the entire blood passing space 2. The shell-and-tube exchanger which is incapable of effecting exchange of heat uniformly on the whole blood being passed therethrough hardly deserves high esteem because it has the drawback of impairing the uniformity of blood temperature distribution, suffering the exchange of heat on the blood to proceed excessively or insufficiently, and bringing about adverse effects upon the blood components.

Further, the conventional heat exchanger is so constructed that a heat-exchanging medium inlet port for introducing the heat-exchanging medium into the heat exchanger and a heat-exchanging medium outlet port for discharging the heat-exchanging medium from within the heat exchanger are integrally formed with a housing of the heat exchanger and are fixed on the housing. A connection tube which leads out of a heat-exchanging medium temperature controller communicating with the heat-exchanging medium inlet port and the heat-exchanging medium outlet port is generally large in diameter and hard to the touch. The connection between the coupler disposed on the heat-exchanging medium inlet port or the heat-exchanging medium outlet port and the coupler disposed at the leading end of the connection tube of the heat-exchanging medium temperature controller is obtained only with difficulty. Moreover, there is a risk that this connection will be disrupted in consequence of a deviation of the positional relationship between the heat exchanger and the heat-exchanging medium temperature controller during the course of operation.

An object of this invention, therefore, is to provide an improved heat exchanger for medical treatment.

Another object of this invention is to provide a heat exchanger for medical treatment which, in the extracorporeal blood circulation, enables the blood drawn out of a patient's body to be kept at a desired temperature.

Yet another object of this invention is to provide a heat exchanger for medical treatment which effects uniform exchange of heat between the blood and the heat-exchanging medium and inflicts damage sparingly on the blood under treatment.

Still another object of this invention is to provide a heat exchanger for medical treatment which enjoys exceptional compactness of design and suffers from only a small pressure loss during the introduction of blood.

A further abject of this invention is to provide a heat exchanger for medical treatment which is capable of being integrated with an oxygenator.

Still further object of this invention is to provide a heat exchanger for medical treatment which excels in operability and ensures great safety.

As claimed the heat exchanger for blood of the present invention comprises a substantially cylindrical housing having a heat- exchanging medium inlet tube disposed at one end portion thereof, a heat-exchanging medium outlet tube disposed at another end portion thereof, a blood inlet tube and a blood outlet tube disposed at circumferential portions between said medium inlet tube and said medium outlet tube and extending in directions substantially perpendicular to the longitudinal axis of said housing;

A multiplicity of heat-exchanging cylindrical tubes are distributed cross-sectionally throughout the entire interior of the cylindrical housing and housed within said housing so as to extend in the longitudinal direction thereof, each of said heat-exchanging tube defining therein, an inner space for passing the heat-exchanging medium;

A pair of partition walls are secured to the inner wall of said housing at locations neighboring the opposite end parts thereof so as to form a pair of heat-exchanging medium chambers which respectively communicate with said medium inlet tube and said medium outlet tube, said partition walls respectively supporting thereon end portions of said heat-exchanging tubes to allow said heat-exchanging tubes to be separated from each other so as to define in said housing a blood chamber which communicates with said blood inlet tube and said blood outlet tube, said partition walls allowing said inner spaces of the heat-exchanging tubes to communicate with said heat-exchanging medium chambers.

According to the present invention, said blood inlet tube and said blood outlet tube extend in directions substantially tangent to the outer circumferential surface of said housing so that the blood in said blood chamber comes into uniform contact with substantially all the heat-exchanging tubes.

This invention also discloses a heat exchanger for medical treatment which is produced by disposing a multiplicity of heat-exchanging tubes mutually separated inside a cylindrical housing possessing closed opposite ends in the longitudinal direction of the housing, partition wall disposed at the opposite end parts of the heat-exchanging tubes to hold the heat-exchanging tubes fast watertightly on the lateral wall of the housing without closing the openings of the heat-exchanging tubes and, at the same time, partition the interior of the housing into three spaces, a blood inlet tube and a blood outlet tube severally extended inwardly from outside substantially along straight lines perpendicular to the longitudinal direction of the housing and tangent to the peripheral surface of the housing to communicate with a blood passing space formed in the central part of the housing by the two partition walls, the lateral wall of the housing, and the outer walls of the heat-exchanging tubes, and a heat-exchanging medium inlet tube to communicate with one of two heat-exchanging medium passing spaces formed at the end parts of the housing communicating with the inner spaces of the heat-exchanging tubes watertightly separated from the blood passing space and a heat-exchanging medium passing space. This invention further discloses a heat exchanger for medical treatment wherein the blood inlet tube communicates with the blood passing space in the proximity of one of the partition walls and the blood outlet tube communicates with the blood passing space in the proximity of the other partition wall. This invention also discloses a heat exchanger for medical treatment wherein the blood inlet tube and the blood outlet tube assume a positional relation such that they are mounted at about 180° from each other around the peripheral surface of the blood passing space.

The objects of this invention described above are accomplished by a heat exchanger for medical treatment comprising a cylindrical blood passing space and a multiplicity of heat-exchanging tubes disposed inside the blood passing space in the longitudinal direction of the blood passing space and provided with an inner space watertightly separated from the blood passing space and effecting exchange of heat between the blood being passed through the blood passing space and a heat-exchanging medium being passed through the inner spaces of the heat-exchanging tubes through the medium of walls of the heat-exchanging tubes, characterized by the fact that a blood inlet tube for introducing blood into the blood passing space and a blood outlet tube for discharging blood from the blood passing space are severally extended inwardly from outside substantially along straight lines perpendicular to the longitudinal direction of the blood passing space and tangent to the peripheral place of the blood passing space and allowed to communicate with the blood passing space and the heat-exchanging tubes are disposed uniformly separated mutually throughout the entire blood passing space except for an empty space portion formed by extending in the axial direction of the blood passing space a portion enclosed in the shape of a bow possessing a chord substantially parallel to and equal to or slightly longer than a line segment connecting two points of intersection between two inner peripheral lines of the blood inlet tube and the circumference of the blood passing space in a cross section perpendicular to the axis of the blood passing space inclusive of the axial line of the blood inlet tube and an empty space portion formed by extending in the axial direction of the blood passing space a portion enclosed in the shape of a bow possessing a chord substantially parallel to and equal to or slightly longer than a line segment connecting two points of intersection between two inner peripheral lines of the blood outlet tube and the circumference of the blood passing space in a cross section perpendicular to the axis of the blood passing space inclusive of the axial line of the blood output tube.

This invention further discloses a heat exchanger for medical treatment which is produced by disposing a multiplicity of heat-exchanging tubes mutually separated inside a cylindrical housing possessing closed opposite ends in the longitudinal direction of the housing, partition walls disposed at the opposite end parts of the heat-exchanging tubes to hold the heat-exchanging tubes fast watertightly on the lateral wall of the housing without closing the openings of the heat-exchanging tubes and, at the same time, partition the interior of the housing into three spaces, a blood inlet tube and a blood outlet tube to communicate with a blood passing space formed in the central part of the housing by the two partition walls, the lateral wall of the housing, and the outer walls of the heat-exchanging tubes, and a heat-exchanging medium inlet tube communicating with one of two heat-exchanging medium passing spaces formed at the end parts of the housing communicating with the inner spaces of the heat-exchanging tubes watertightly separated from the blood passing space and a heat-exchanging medium outlet tube communicating with the other heat-exchanging medium passing space. This invention also discloses a heat exchanger for medical treatment wherein the blood inlet tube communicates with the blood passing space in the proximity of one of the partition walls and the blood outlet tube communicates with the blood passing space in the proximity of the other partition wall. This invention further discloses a heat exchanger for medical treatment wherein the blood inlet tube and the blood outlet tube assume a positional relation such that they are mounted at about 180° from each other around the peripheral surface of the blood passing space. Further, this invention discloses a heat exchanger for medical treatment wherein the two empty space portions destitute of arrangement of heat-exchanging tubes within the blood passing space occupy a total volume of less than 40% of the blood passing space.

4

The objects of this invention described above are accomplished by a heat exchanger for medical treatment comprising a cylindrical housing possessing a blood inlet and a blood outlet, a multiplicity of slender heat-exchanging tubes accommodated within the cylindrical housing, partition walls adapted to fix the opposite end parts of the slender heat-exchanging tubes watertightly to the cylindrical housing and partition the interior of the housing into a blood chamber communicating with the blood outlet and the blood inlet and a heat-exchanging medium chamber formed by the tube interiors, and a medium inlet and a medium outlet communicating with the heat-exchanging medium chamber, which comprises at least one rib formed on the inner wall surface of the housing as extended parallelly to the slender tubes opposed to the inner wall surface and adapted to retard the flow of blood between the slender tubes and the inner wall surface of the housing. This heat exchanger is desired to possess a space destitute of the slender tubes near the position of the housing at which the blood inlet is located. Optionally, the blood inlet may be fitted as directed toward the inner wall side of the housing at a stated angle from the center of the housing. The heat exchanger is desired to possess a space destitute of the slender tubes near the position of the housing at which the blood outlet is located. Optionally, the blood outlet may be fitted as directed toward the inner wall side of the housing at a stated angle from the center of the housing. Further, the blood inlet is desired to be fitted as directed toward the inner wall side of the housing at a stated angle from the center of the housing and the ribs to be formed on the inner surface of the housing toward which the blood inlet is directed. Further, the blood outlet may be disposed substantially parallelly to the direction of the blood inlet. Further, multiplicity of slender tubes are desired to be accomodated as separated mutually by a substantially uniform distance inside the housing and the distance between the ribs and the slender tubes approximating the ribs to be smaller than the distance between the slender tubes. Further, the ribs are desired to be disposed near those of the slender tubes which are located in the neighborhood of the blood inlet. The housing is formed in a substantially cylindrical shape, for example. The heat exchanger may be provided with a plurality of ribs.

The objects of this invention described above are further accomplished by a heat exchanger for medical treatment which comprises an integrally molded cylindrical housing possessing a heat-exchanging medium inlet port disposed at one end part thereof, a heat-exchanging medium outlet port disposed at the other end part thereof, and a blood inlet port and a blood outlet port disposed at positions between the medium inlet port and the medium outlet port, a multiplicity of slender heat-exchanging tubes accomodated within the housing, partition walls adapted to fix the opposite end parts of the slender tubes watertightly to the cylindrical housing and partition the interior of the housing into a blood chamber communicating with the blood outlet port and the blood inlet port and a heat-exchanging medium chamber formed inside the tubes and adapted to communicate with the medium inlet port and the medium outlet port, and sealing members serving to seal the opposite end parts of the housing.

The heat exchanger just described is desired to possess a space destitute of the slender tubes near the position of the housing at which the blood inlet port is located. It is further desired to possess a space destitute of the slender tubes near the position of the housing at which the blood outlet port is located. The housing is formed in a cylindrical shape, for example. The partition walls are formed of a potting compound, for example. The partition walls comprise perforated plates adapted to keep hold of the end parts of the slender tubes and a potting compound for watertightly fixing the perforated plates to the slender tubes. The perforated plates are desired each to possess a multiplicity of holes formed in a shape such that the inside diameter of hole at one end is larger than the outside diameter of the slender tubes and at the other end smaller than the outside diameter of the slender tubes. Further, the medium inlet port is desired to be fitted as directed toward the inner surface side of the housing at a stated angle from the center of the housing. Further, the medium inlet port is desired to be fitted in a direction substantially tangent to the outer surface of the housing. Further, the medium inlet port is desired to be fitted in a direction substantially tangent to the outer surface of the housing. The medium outlet port may be fitted as directed toward the inner surface side of the housing at a stated angle from the center of the housing. The medium outlet port, for example, is fitted in a direction substantially tangent to the outer surface of the housing.

The objects of this invention described above are further accomplished by a method for the production of a heat exchanger for the blood under treatment by the extracorporeal circulation, which comprises the steps of forming a cylindrical housing possessing heat-exchanging medium ports disposed each one at the opposite end parts thereof and a blood inlet port and a blood outlet port disposed at positions between the medium ports, attaching a first sealing member at a position between those of the medium ports and the blood ports which are located at one end part of the cylindrical housing, inserting through the other end part of the cylindrical housing into the cylindrical housing a slender tube distibuting device possessing a multiplicity of holes for insertion of slender heat-exchanging tubes and a multiplicity of slender heat-exchanging tubes, attaching a second sealing member at a position between those of the medium ports and the blood ports which are located at the other end part of the cylindrical housing, injecting a potting compound through the blood port on one end part side and the blood port on the other end part side of the cylindrical housing with the end parts of the slender heat-exchanging tubes kept in a closed state thereby forming partition walls for fixing the opposite end parts of the slender heat-exchanging tubes to the cylindrical housing, removing the first sealing member and the second sealing member, and fitting seal members one each to the opposite ends of the cylindrical housing.

The insertion of the slender tube distributing device possessing a multiplicity of holes for insertion of slender heat-exchanging tubes and a multiplicity of slender heat-exchanging tubes through the other end part of the cylindrical housing into the cylindrical housing is carried out, for example, while keeping the cylindrical housing set upright on the one end part of the cylindrical housing to which the first sealing member has been attached. Otherwise, the insertion of the slender tube distributing device possessing a multiplicity of holes for insertion of slender heat-exchanging tubes and a multiplicity of slender heat-exchanging tubes through the other end part of the cylindrical housing into the cylindrical housing is carried out, for example, by a procedure which comprises first inserting through the other end part of the cylindrical housing into the cylindrical housing the slender tube-distributing device possessing a multiplicity of holes for insertion of slender heat-exchanging tubes and subsequently inserting the multiplicity of slender heat-exchanging tubes. The formation of partition walls for fixing the opposite end parts of the slender heat-exchanging tubes to the cylindrical housing by the injection of a potting compound through the blood port on one end part side and the blood port on the other end part side of the cylindrical housing with the end parts of the slender heat-exchanging tubes kept in a closed state is accomplished, for example, by injecting the potting compound through the blood port on one end part side of the cylindrical housing, then turning the cylindrical housing upside down, injecting the potting compound through the blood port on the other end part side of the cylindrical housing, and allowing the injected portions of the potting compound to set and give rise to partition walls. Further, the formation of partition walls for fixing the opposite end parts of the slender heat-exchanging tubes to the cylindrical housing by the injection of a potting compound through the blood port on one end part side and the blood port on the other end part side of the cylindrical housing with the end parts of the slender heat-exchanging tubes kept in a closed state is accomplished, for example, by injecting the potting compound through the blood port on one end part side of the cylindrical housing, allowing the injected portion of the potting compound to set, then removing the second sealing member and the slender tube-distributing device, attaching the second sealing member, injecting the potting compound through the blood port on the other end part side of the cylindrical housing with the end parts of the slender heat-exchanging tubes kept in a closed state, and allowing the injected portion of the potting compound to set. The step of removing the first sealing member and the second sealing member and the step of attaching each seal members to the opposite ends of the cylindrical housing are carried out, for example, by a procedure which comprises first removing the first sealing member and the second sealing member and subsequently attaching each seal members to the opposite end parts of the cylindrical housing. Further, the step of removing the first sealing member and the second sealing member and the step of attaching each seal members to the opposite ends of the cylindrical housing are carried out, for example, by a procedure which comprises a step of removing either the first sealing member or the second sealing member and attaching one of the seal members to the end part from which the sealing member has been removed and a step of removing the remaining sealing member and attaching the remaining seal member to the end part from which the remaining sealing member has been removed. Further, the first sealing member is desired to comprise an elastic sealing member and an assembly retaining device for retaining the elastic sealing member in place.

The objects of this invention described above are further accomplished by a heat exchanger for medical treatment comprising a housing for enclosing a closed empty space therewith and heat-exchanging tubes disposed inside the housing and provided each with an inner space watertightly separated from the closed empty space and effecting exchange of heat between a first fluid passed through the inner spaces of the heat-exchanging tubes and a second fluid passed through the closed empty space of the housing through the medium of walls of the heat-exchanging tubes, which heat exchanger is characterized by the fact that a heat-exchanging medium inlet port and a heat-exchanging medium outlet port for causing the first fluid or the second fluid intended as a heat-exchanging medium to be respectively introduced into and discharged from the inner spaces of the heat-exchanging tubes disposed inside the housing or the closed emtpy space of the housing are extended from the outer wall surface of the housing with flexible tubes.

BRIEF DESCRIPTION OF THE DRAWINGS

Fig. 1 is a cross section taken through a conventional heat exchanger perpendicularly to the axis thereof to illustrate the construction thereof,

Fig. 2 is a cross section taken through the conventional heat exchanger in the directon of the axis thereof,

Fig. 3 is a partially cutaway perspective view illustrating a typical heat exchanger for medical treatment as one embodiment of the present invention,

Fig. 4 is a cross section taken through Fig. 3 along the line IV-IV,

Fig. 5 is a cross section taken through Fig. 3 along the line V-V,

Fig. 6 is a partially sectioned front view of an oxygenator incorporating therein the heat exchanger of the present invention,

Fig. 7 is a perspective view of the oxygenator illustrated in Fig. 6,

Fig. 8 is a partially cutaway perspective view illustrating the construction of a typical heat exchanger as another embodiment of this invention,

Fig. 9 is a cross section taken through Fig.8 along the line IX-IX,

Fig. 10 is a cross section taken through Fig. 8 along the line X-X,

Figs. 11A and 11B are cross sections of still other embodiments,

Fig. 12 is a cross section of yet another embodiment,

Fig. 13 is a cross section of still yet another embodiment,

Fig. 14 is a partially cutway front view illustrating the another embodiment of the heat exchanger of the present invention,

Fig. 15 is a partially enlarged cross section or Fig. 14,

Fig. 16 is a drawing for explaining the manufacturing process of the heat exchanger in accordance with the present invention,

Fig. 17 is a partially enlarged cross section of Fig. 16, and

Fig. 18 is a perspective view of still another embodiment of the heat exchanger of the present invention.

DESCRIPTION OF THE PREFERRED EMBODIMENTS

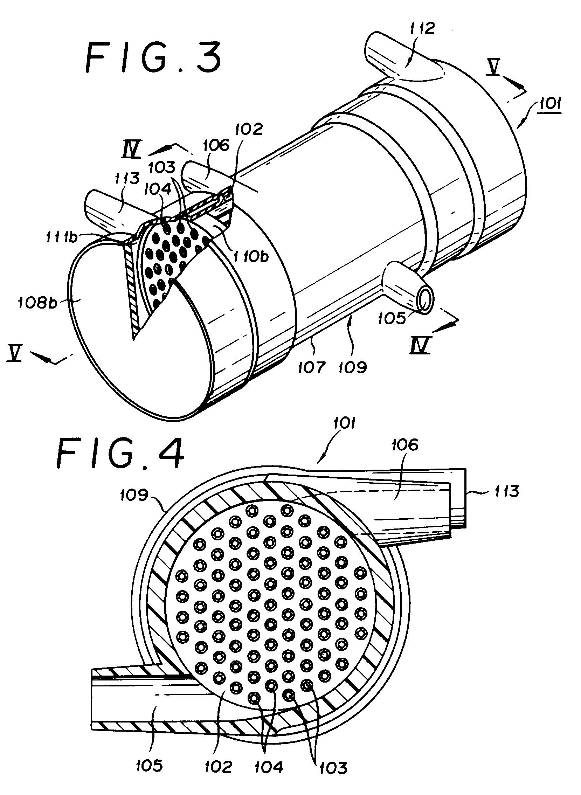

A heat exchanger 101 of this invention for medical treatment, as illustrated in Figs. 3 to 5, comprises a cylindrical blood passing space 102 and a multiplicity of heat-exchanging tubes 104 disposed inside the cylindrical blood passing space 102 along the longitudinal direction of the blood passing space 102 and each provided with an inner space watertightly separated from the blood passing space 102 and effects exchange of heat across the walls of the heat-exchanging tubes 104 between the blood passed through the blood passing space 102 and a heat-exchanging medium passed. through the inner spaces 103 of the heat-exchanging tubes 104. This heat exchanger 101 has as the salient characteristic thereof the fact that a blood inlet tube 105 for introducing blood into the blood passing space 102 and a blood outlet tube 106 for discharging the blood from the blood passing space 102 are severally extended inwardly from outside substantially along straight lines perpendicular to the longitudinal direction of the blood passing space 102 and tangent to the peripheral surface of the blood passing space 102 and allowed to communicate with the blood passing space 102.

When the blood inlet tube 105 and the blood outlet tube 106 are severally extended inwardly from outside substantially along straight lines perpendicular to the longitudinal direction of the blood passing space 102 and allowed to communicate with the blood passing space 102 as described above, the blood introduced through the blood inlet tube 105 is induced to assume a flow tending to revolve inside the blood passing space 102 along the periphery of the blood passing space 102 and the blood passed through the interior of the blood passing space 102 is caused to come into uniform contact with substantially all the heat-exchanging tubes 104 disposed inside the blood passing space 102 except mainly for a specific group of heat-exchanging tubes 104 which are located in the central part of the blood passing space 102, namely on a roughly straight line connecting the communicating entrance to the blood inlet tube 105 and the communicating entrance to the blood outlet tube 106. Thus, the exchange of heat is attained uniformly within the blood passing space 102 without entailing any locally excessive or insufficient exchange of heat. Further in the heat exchanger of this invention, since the blood is passed outside the heat-exchanging tubes 105, it is neither compelled to find only a limited flow path during the course of introduction thereof into the blood passing space 102 nor forced to suffer from any large pressure loss or entail any heavy damage to the blood components.

The heat exchanger for medical treatment in the present embodiment is so constructed that inside a cylindrical housing 109 of closed opposite ends formed of a housing proper 107 and end plates 108a, 108b closing the open opposite ends thereof as illustrated in Figs. 3 to 5, the multiplicity of heat-exchanging tubes 104 are disposed mutually separated along the longitudinal direction of the housing 109 and partition walls 110a, 110b disposed each one at the opposite end parts of the plurality of heat-exchanging tubes 104 retain the heat-exchanging tubes 104 watertightly to the lateral wall of the housing 109 without closing the openings of the heat-exchanging tubes 104. At the same time, these partition walls 110a, 110b serve the purpose of partitioning the interior of the housing 109 into three empty spaces. The central portion of the housing 109 enclosed with the two partition walls 110a, 110b, the lateral wall of the housing 109, and the outer walls of the heat-exchanging tubes 104 constitutes itself the blood passing space 102 and the two end portions of the housing 109 watertightly separated from the blood passing space 102 and enclosed with the partition walls 110a, 110b and the end part walls and the lateral wall of the housing 109 constitute themselves the heat-exchanging medium passing spaces 111a, 111b. These two heat-exchanging medium passing spaces 111a, 111b both communicate with the inner spaces 103 of the heat-exchanging tubes 104 which are watertightly separated from the blood passing space 102. In the construction formed as described above, the blood inlet tube 105 and the blood outlet tube 106 are allowed to communicate with the blood passing space 102 and the heat-exchanging medium outlet tube 112 is allowed to communicate with the heat-exchanging medium passing space 111a and the heat-exchanging medium inlet tube 113 with the other heat-exchanging medium passing space 111b.

Further, in the heat exchanger 101 of the present embodiment for medical treatment, the blood inlet tube 105 and the blood outlet tube 106 are severally extended inwardly from outside substantially along straight lines perpendicular to the longitudinal direction of the housing 109 and tangent to the peripheral surface of the housing 109, namely substantially along straight lines perpendicular to the longitudinal direction of the blood passing space 102 and tangent to the peripheral surface of the blood passing space 102, and consequently allowed to communicate with the blood passing space 102. The blood inlet tube 105 and the blood outlet tube 106 which are extended inwardly from outside need not fall exactly on the straight lines perpendicular to the longitudinal direction of the blood passing space 102 and tangent to the peripheral surface of the blood passing space 102 but may deviate from the straight lines to an extent such that they will not be prevented from effectively imparting to the blood passed through the blood passing space 102 a flow along the peripheral surface of the blood passing space 102. Further, the blood inlet tube 105 and the blood outlet tube 106 are only required, at least in the portions thereof immediately before their points of communication with the blood passing space 102, to run roughly along the straight lines perpendicular to the longitudinal direction of the blood passing space 102 and tangent to the peripheral surface of the blood passing space 102. For the subsequent portions thereof, the direction is a matter for arbitrary decision. The positions at which the blood inlet tube 105 and the blood outlet tube 106 are to be located are not specifically defined. For the purpose of ensuring effective exchange of heat between the heat-exchanging medium passed through the inner spaces of the heat-exchanging tubes 104 inserted inside the blood passing space 102 and the blood passed through the blood passing space 102, they must be allowed to communicate with the blood passing space 102 at mutually separated positions. Desirably, as illustrated in Figs. 3 to 5, the blood inlet tube 105 is allowed to communicate with the blood passing space 102 near one of the partition walls 111a and 111b and the blood outlet tube 106 to communicate with the blood passing space 102 near the other partition walls 111b or 111a. Further, the blood inlet tube 105 and the blood outlet tube 106 are desired to assume a positional relation such that they are mounted at about 180° from each other around the peripheral surface of the blood passing space 102 as illustrated in Figs. 3 and 4.

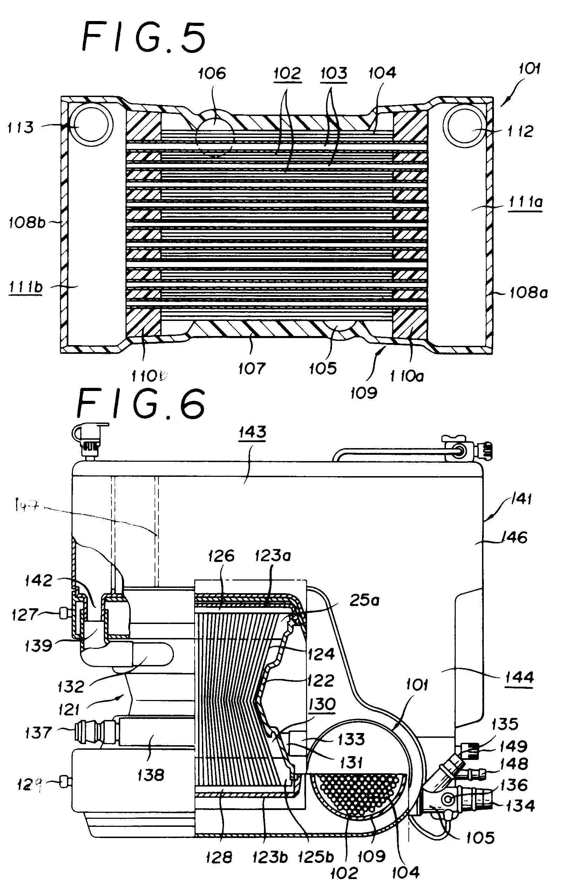

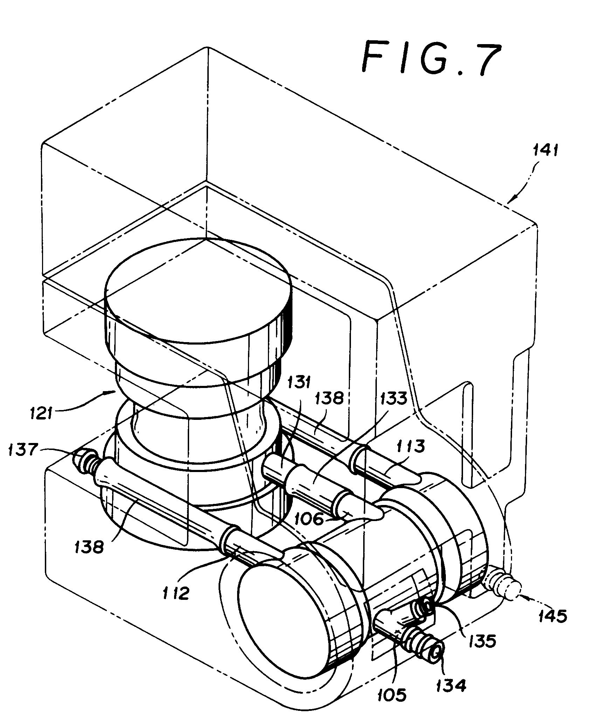

The heat exchanger 101 for medical treatment of the present embodiment which is constructed as described above is put to use as incorporated suitably in a varying extracorporeal circulation circuit. Since it is featured by exceptional compactness of design and high performance, it can be advantageously utilized as integrated with an oxygenator and a blood storing tank and operated as an oxygenator system as illustrated in Figs. 6 and 7, for example.

In the embodiment illustrated in Figs. 6 and 7, an oxygenator 121 is provided with a housing which comprises a cylindrical housing body 122 and fitting covers 123a, 123b closing the open opposite end parts of the housing body 122. Inside the housing, a multiplicity of hollow fiber membranes 124 are paralelly disposed mutually separated along the longitudinal direction of the housing and distributed cross-sectionally throughout the entire interior of the housing. The opposite end parts of these hollow fiber membranes 124 are watertightly retained on the housing body 122 by means of partition walls 125a, 125b, with the openings of the opposite end parts kept in an opened state. A gas inlet port 127 is disposed so as to communicate with a gas inlet space 126 defined by the fitting cover 123a, the housing body 122, and the partition wall 125a and allowed to communicate with the inner spaces of the hollow fiber membranes and a gas passing port 129 is disposed so as to communicate with a gas passing space 128 defined by the other fitting cover 123b, the housing body 122, and the partition wall 125b and allowed to communicate with the inner spaces of the hollow fiber membranes. Further, a blood inlet tube 131 and a blood outlet tube 132 are disposed so as to communicate with a blood chamber 130 formed of the inner wall of the housing body 122, the two partition walls 125a, 125b, and the outer walls of the hollow fiber membranes 124.

The oxygenator 121 illustrated in the present embodiment is of a type which effects exchange of gas by blowing an oxygen-containing gas such as air into the inner spaces of the hollow fiber membranes and passing blood outside the hollow fiber membranes 114. Otherwise, the oxygenator may be constructed in a type which effects the exchange of gas by passing blood in the inner spaces of the hollow fiber membranes and a passing the oxygen-containing gas outside the hollow fiber membranes. Alternatively, an oxygenator of a type using flat gas-exchange membranes is also usable. In all these types of oxygenator, particularly desirable is the type which passes the blood outside the hollow fiber membranes as illustratd in the present embodiment. Since the oxygenator of this type has any sparing pressure loss, the oxygenator system using this oxygenator does not need a blood pump in front of the oxygenator inserted in the path of circulation circuit. It has only to rely upon the removal of blood from a patient's body owing to the head of pressure to obtain required flow of the blood to the oxygenator and further to the blood storing tank.

The blood outlet tube 106 of the heat exchanger 101 for medical treatment is allowed to communicate watertightly with the blood inlet tube 131 of the oxygenator 121 through the medium of a connection tube 133 as illustrated in Fig. 7. The watertight connection of the connection tube 133 to the blood inlet tube 131 of the oxygenator 121 and to the blood outlet tube 6 of the heat exchanger 101 for medical treatment is attained by tight fitting by the use of a screw, a taper, or an O-ring or by tight adhesion by means of ultrasonic waves or high-frequency waves or by with adhesive agent. Of course, it is permissible to connect the blood inlet tube 131 of the oxygenator 121 directly and watertightly to the blood outlet tube 6 of the heat exchanger 101 for medical treatment by similar means of connection. The exchanger for medical treatment in the present embodiment and the one illustrated in Figs. 3 to 5 are substantially identical, though they differ slightly with respect to the positions at which the blood inlet tube 105 and the blood outlet tube are located. The blood inlet tube 105 in the heat exchanger 101 for medical treatment in the present embodiment is provided with a cardiotomy inlet port 135 for introduction of the blood shed during the course of surgical operation in addition to a blood inlet port 134 for connection to the extracorporeal circulation path. The blood inlet tube 105 of this heat exchanger 101 is provided with a temperature measuring probe insertion hole 136. To the heat-exchanging medium inlet tube 113 and the heat-exchanging medium outlet tube 112, flexible extension tubes 138 provided at the leading end part thereof with a water inlet port (not shown) or a water outlet port 137 are connected.

In the meantime, a blood inlet 142 of a blood storing tank 141a is connected watertightly with a connection tube 139 to the blood outlet tube 132 of the oxygenator 121. The watertight connection of the connection tube 139 to the blood outlet tube of the oxygenator 121 and to the blood outlet tube 142 of the blood storing tank 141 is attained in the same manner as in the watertight connection of the connection tube 133 to the blood inlet tube 131 of the oxygenator 121 and to the blood outlet tube 106 of the heat exchanger 101 for medical treatment.

With a housing 146 made of rigid material and provided with a blood inlet 142, a blood inlet part 143 communicating with the blood inlet 142 and possessing a bottom surface having substantially no head of pressure from the blood inlet 142, a blood storing part 144 communicating with the blood inlet part 143 and possessing a bottom surface destined to fall gradually from the blood inlet part 143, and a blood outlet 145 disposed below the blood storing part 144, the blood storing tank 141 which is connected to the oxygenator 121 is formed by disposing a defoaming member 147 across the entire width of the blood flow path of the blood inlet part 143. This blood storing tank 141 is provided not only with the blood outlet 145 connected to the extracorporeal circulation path but also with a cardio-pregear part 148 destined to be connected to the path for forwarding blood to the cardiac vein and adapted to communicated with the lower part of the blood storing part 144. It is further provided a temperature measuring probe insertion port 149 intended to measure the temperature of blood within the blood storing tank 141.

In the oxygenator system which integrates the heat exchanger 101 for medical treatment and the blood storing tank 114 with the oxygenator 121 as described above, the blood withdrawn from a patient's body flows into the heat exchanger 101 via the blood inlet tube 105. Since the blood inlet tube 105 and the blood outlet tube 106 are severally extended inwardly from outside substantially along the straight lines perpendicular to the longitudinal direction of the blood passing space 102 and tangent to the peripheral surface of the blood passing space 2 and allowed to communicate with the blood passing space 102 as described above, the blood introduced through the blood inlet tube 105 into the blood passing space 102 is induced to generate a flow tending to revolve inside the blood passing space 102 along the inner peripheral surface of the blood passing space 102 and the blood passed through the interior of the blood passing space 102 is brought into uniform contact with substantially all the heat-exchanging tubes 104 disposed inside the blood passing space 102, except mainly for the specific group of heat-exchanging tubes 104 which are located in the central part of the blood passing space 102, namely on the roughly straight line connecting the point of communication with the blood inlet tube 105 and the point of communication with the blood outlet tube 106. As a result, the exchange of heat across the walls of the heat-exchanging tubes within the blood passing space 102 between the blood and the water, i.e. a heat-exchanging medium, which is introduced through the heat-exchanging medium inlet tube 113 into the heat-exchanging medium passing space 111a, passed through the inner spaces 103 of the heat-exchanging tubes 104 to the heat-exchanging medium passing space 111b, and discharged through the heat-exchanging medium outlet tube 112 is carried out efficiently and uniformly. The blood which has been heated or cooled to a desired temperature is led out of the heat exchanger 101 through the blood outlet tube 106 and then forwarded to the oxygenator 121 through the blood inlet tube 131 of the oxygenator 121 communicating watertightly with the blood outlet tube 106. The blood which has flowed into the oxygenator 121 via the blood inlet tube 131, while flowing through the blood chamber 130, exchanges gas through the medium of the walls of the hollow fiber membranes 124 with the oxygen-containing gas which is flowing through the inner spaces of the hollow fiber membranes 124. Consequently, the blood is divested of excess carbon dioxide gas and instead replenished with fresh oxygen. The blood which has been oxygenated flows out of the blood outlet tube 132 of the oxygenator 121 and then continues its flow into the blood storing tank 141 through the blood inlet 142 of the blood storing tank 141. The blood which has flowed from the blood inlet 142 to the blood inlet part 143 communicating therewith and reached the defoaming member 147 disposed in route to the blood inlet part 143 is defoamed during the course of its passage through the defoaming member 147 by the fact that the bubbles entrained by the blood come into contact with the foam cells of the defoaming member 147, gradually agglomerate, and depart from the blood and move to the upper empty space inside the blood storing tank 141. The defoamed blood further moves to the blood storing part 144, remains temporarily in the blood storing part 144, releases itself through the blood outlet 145 disposed below the blood storing part 144, and finds its way to the patient's body.

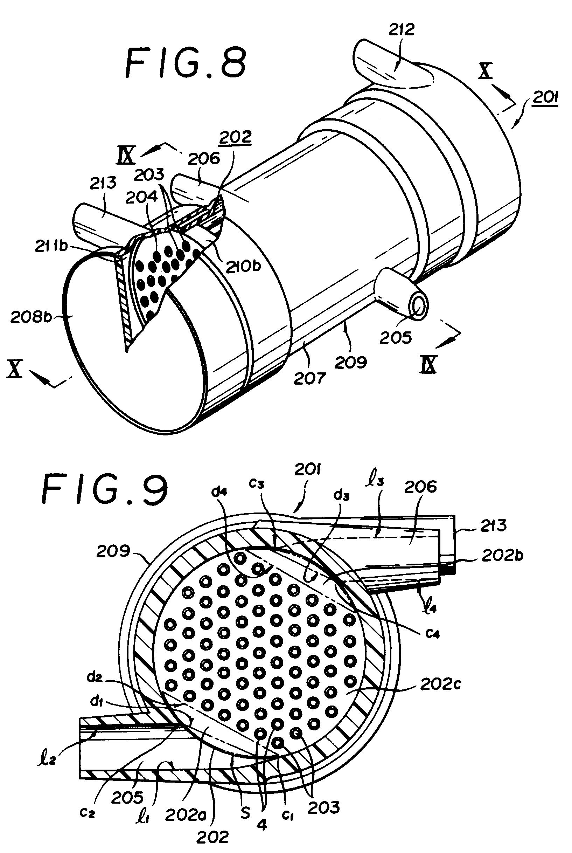

Figs. 8 to 10 illustrate another embodiment of this invention. This embodiment, similarly to that illustrated in Figs. 3 to 5, comprises a heat exchanger 201 for medical treatment which comprises a cylindrical blood passing space 202 and a multiplicity of heat-exchanging tubes 204 disposed inside the cylindrical blood passing space 202 along the longitudinal direction of the blood passing space and provided each with an inner space 203 watertightly separated from the blood passing space 202 and effects exchange of heat across the walls of the heat-exchanging tubes 204 between the blood passed through the blood passing space 202 and the heat-exchanging medium passed through the inner spaces 203 of the heat-exchanging tubes 204. This heat exchanger 201 has as the salient characteristic that a blood inlet tube 205 for introducing blood into the blood passing space 202 and a blood outlet tube 206 for discharging the blood from the blood passing space 202 are severally extended inwardly from outside substantially along straight lines perpendicular to the longitudinal direction of the blood passing space 202 and tangent to the peripheral surface of the blood passing space 202 and the heat-exchanging tubes 204 are disposed as uniformly separated mutually throughout the entire blood passing space 202 except for an empty space portion 202a formed by extending in the axial direction of the blood passing space 202 a portion enclosed in the shape of a bow possessing a chord $d_2$ substantially parallel to and equal to or slightly longer than a line segment $d_1$ connecting two points $C_1$, $C_2$ of intersection between two inner peripheral lines $\ell_1$, $\ell_2$ of the blood inlet tube 205 and the circumferences of the blood passing space in a cross section perpendicular to the axis of the blood passing space inclusive of the axial line of the blood inlet tube 205 and an empty space portion 202b formed by extending in the axial direction of the blood passing space a portion enclosed in the shape of a bow possessing a chord $d_4$ substantially parallel to and equal to or slightly longer than a line segment $d_3$ connecting two points $C_3$, $C_4$ of intersection between two inner peripheral lines $\ell_3$, $\ell_4$ of the blood outlet tube 206 and the circumference S of the blood passing space 202 in a cross section perpendicular to the axis of the blood passing space inclusive of the axial line of the blood outlet tube 206.

When the blood inlet tube 205 and the blood outlet tube 206 are severally extended inwardly from outside substantially along the straight lines perpendicular to the longitudinal direction of the blood passing space 202 and tangent to the peripheral surface of the blood passing space 202 and allowed to communicate with the blood passing space 202 as described above, the blood introduced through the blood inlet tube 205 is induced to generate a flow tending to revolve inside the blood passing space 202 along the peripheral surface of the blood passing space and the opening through which the blood inlet tube 205 communicates with the blood passing space 202 grows in size. Further, an empty space destitute of heat-exchanging tubes is present in front of the opening for communication. As the result, the blood introduced through the blood inlet tube 205 flows substantially uniformly throughout the entire blood passing space and comes into uniform contact with substantially all the heat-exchanging tubes 204 disposed within the blood passing space 202 except mainly for the specific group of heat-exchanging tubes 204 located in the central part of the blood passing space 202, namely on the roughly straight line connecting the point of communication with the blood inlet tube 205 and the point of communication with the blood outlet tube 206. By the same token, since the opening for communication of the blood outlet tube 206 with the blood passing space 202 grows in size and an empty space destitute of heat-exchanging tubes 204 is present in front of the opening for communication, the blood flowing through any portion within the blood passing space 202 is discharged substantially uniformly out of the blood outlet tube 206. As a result, the exchange of heat within the blood passing space 202 can be carried out uniformly without entailing any locally excessive or insufficient exchange of heat. Further, in the heat exchanger of the present embodiment, since the blood flows outside the heat-exchanging tubes 204 and the opening for communication of the blood inlet tube 205 with the blood passing space 202 grows in size and an empty space destitute of heat-exchanging tubes 204 is present in front of the opening for communication, the blood suffers no heavy pressure loss during the course of introduction thereof into the blood passing space 202 and entails infliction of only sparing damage of the blood components.

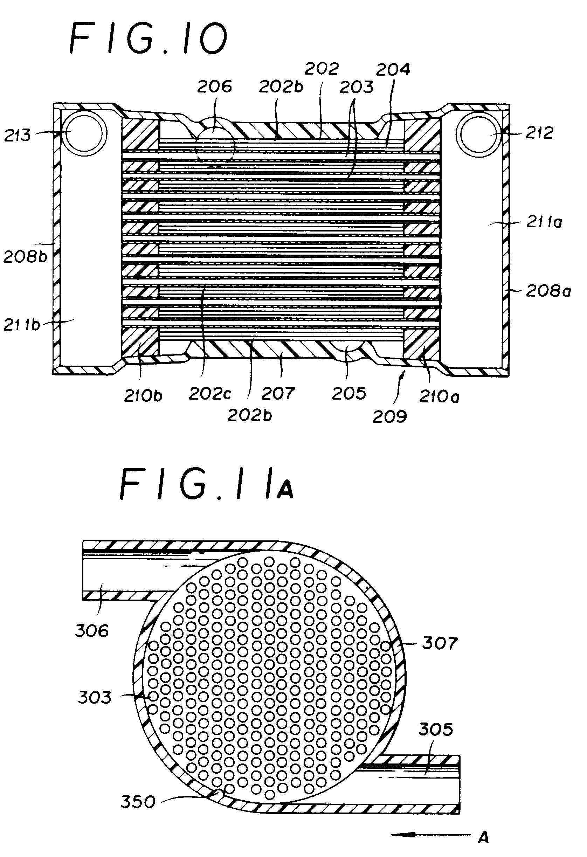

Fig. 10 is a cross section in the axial direction of the present embodiment.

The heat exchanger for medical treatment of the present embodiment is constructed so that, inside a cylindrical housing 209 comprising a housing proper 207 and end plates 208a, 208b closing the open opposite end parts of the housing proper 207, a multiplicity of heat-exchanging tubes 204 are disposed mutually separated along the longitudinal direction of the housing 207 and partition walls 210a, 210b disposed in the opposite end parts of the plurality of heat-exchanging tubes 204 retain the heat-exchanging tubes 204 watertightly to the lateral wall of the housing 209 without closing the openings of the heat-exchanging tubes 204. The partition walls 210a, 210b further serve the purpose of partitioning the interior of the housing 209 into three empty spaces. Specifically, the central portion of the housing 209 enclosed with the two partition walls 210a, 210b, the lateral wall of the housing 209, and the outer walls of the heat-exchanging tubes 204 constitutes itself the blood passing space 202 and the two end portions of the housing separated watertightly from the blood passing space 202 and enclosed with the partition walls 210a and the partition walls 210b and the end part walls and the lateral wall of the housing 209 constitute themselves the heat-exchanging medium passing spaces 211a, 211b. These two heat-exchanging medium passing spaces 211a, 211b both communicate with the inner spaces of the heat-exchanging tubes 204 which are watertightly separated from the blood passing space 207. The blood inlet tube 205 and the blood outlet tube 206 communicate with the blood passing space 202 which is constructed as described above. Further, a heat-exchanging medium outlet tube 212 is caused to communicate with the heat-exchanging medium passing space 211a and the heat-exchanging medium inlet tube 213 to communicate with the other heat-exchanging medium passing space 211b.

In the heat exchanger 201 for medical treatment of the present embodiment, the blood inlet tube 205 and the blood outlet tube 206 are severally extended inwardly from outside substantially along the straight lines perpendicular to the longitudinal direction of the housing and tangent to the peripheral surface of the housing 209, namely severally extended inwardly from outside substantially along the straight lines perpendicular to the longitudinal direction of the blood passing space 202 and tangent to the peripheral plane of the blood passing space 202, and allowed to communicate with the blood passing space 202. The blood inlet tuba 205 and the blood outlet tube 206 extended inwardly from outside need not fall exactly on the straight lines perpendicular to the longitudinal direction of the blood passing space 202 and tangent to the peripheral plane of the blood passing space 202 but may deviate from the straight lines to a slight extent such that they will not be prevented from effectively imparting to the blood passed through the blood passing space 202 a flow along the peripheral plane of the blood passing space 202. Further, the blood inlet tube 205 and the blood outlet tube 206 are required, at least in the portions thereof immediately before their points of communication with the blood passing space 202, to run roughly along the straight lines perpendicular to the longitudinal direction of the blood passing space 202 and tangent to the peripheral plane of the blood passing space 202. For the subsequent portions thereof, the direction is a matter of arbitrary decision. The positions at which the blood inlet tube 205 and the blood outlet tube 206 are located are not specifically defined. To ensure effective exchange of heat between the heat-exchanging medium passed through the inner spaces of the heat-exchanging tubes 204 inserted inside the blood passing space 202 and the blood passed through the interior of the blood passing space 202, they are required to communicate with the blood passing space 202 at mutually separated positions. Desirably, as illustrated in Fig. 8 and Fig. 10, the blood inlet tube 205 communicates with the blood passing space 202 near either of the partition walls 210a, 210b and the blood outlet tube 206 communicates with the blood passing space 202 near the other partition wall 210b or 210a. Further, the blood inlet tube 205 and the blood outlet tube 206 are desirably assuming a positional relation such that they are mounted at an angle of about 180° from each other around the peripheral surface of the blood passing space 202 as illustrated in Fig. 8 and Fig. 9.

Moreover in the heat exchanger 201 for medical treatment of the present embodiment, the heat-exchanging tubes 204 disposed along the longitudinal direction of the blood passing space 202 are distributed uniformly separated mutually throughout the entire blood passing space 202 except for the empty space portion 202a formed by extending in the axial direction of the blood passing space 202 the portion enclosed in the shape of a bow possessing the chord $d_2$ substantially parallell to and equal to or slightly longer than the line segment $d_2$ connecting the two points $C_1$, $C_2$ of intersection between the two inner peripheral lines $\ell_1$, $\ell_2$ of the blood inlet tube 205 and the circumference S of the blood passing space in the cross section perpendicular to the axis of the blood passing space inclusive of the axial line of the blood inlet tube 202 and the empty space portion 202b formed by extending in the axial direction of the blood passing space 202 the portion enclosed in the shape of a bow possessing regulating chamber for roughly uniformizing the flow volume of the blood with respect to the heat-exchanging tubes 204 disposed within the blood passing space 202.

The empty space portion 202a which functions as a blood flow regulating chamber is given a shape formed by extending in the axial direction of the blood passing space 202 the portion enclosed in the shape of a bow possessing the chord $d_2$ substantially parallel to and equal to or slightly longer than the line segment $d_1$ connecting the points $C_1$, $C_2$ of the blood inlet tube 205 and the circumference S of the blood passing space in the cross section perpendicular to the axis of the blood passing space inclusive of the axial line of the blood inlet tube 205 and the empty space portion 202c is similarly given a shape formed by extending in the axial direction of the blood passing space 202 the portion enclosed in the shape of a blow possessing the chord $d_4$ substantially parallel to and equal to or slightly longer than the line segment $d_3$ connecting the two points $C_3$, $C_4$ of intersection between the two inner circumferential lines $\ell_3$, $\ell_4$ of the blood outlet tube 206 and the circumference S of the blood passing space 202 in the cross section perpendicular to the axis of the blood passing space inclusive of the axial line of the blood outlet tube 206. These special shapes have been adopted for the following reason. If the empty space portions 202a, 202b are excessively slanted relative to the openings for communication of the blood inlet tube 205 and the blood outlet tube 206 with the blood passing space 202, namely if they are given shapes formed by extending in the axial direction of the blood passing space 202 the portion enclosed in the shape of a bow possessing a chord of a widely different inclination from the line segments $d_1$ and $d_3$, they have the drawback of not only narrowing excessively the empty space portion 202c destined to accommodate the heat-exchanging tubes 204 but also impairing the uniformity of blood flow distribution. Further, if the chords $d_2$ and $d_4$ are much longer than the line segments $d_1$ and $d_3$ (equaling the inside diameters of the openings for communication of the blood inlet tube 205 and the blood outlet tube 206 with the blood passing space 202), they have the drawback of excessively narrowing the empty space portion 202c destined to accommodate the heat-exchanging tubes. The chords $d_2$, $d_4$ of the bows which are cross sections of the empty space portions 202a, 202b need not be exactly parallel to the line segments $d_1$, $d_3$ but may be different in inclination from the line segments to a slight extent such that they will not be prevented from substantially uniformly distribution the blood flow in the circumferential direction of the blood passing space 202 in front of the openings for communication of the blood inlet tube 205 and the blood outlet tube 206 with the blood passing space 202. For the purpose of forming the empty space portions 202a, 202b effectively, the chords $d_2$, $d_4$ must be at least equal in length to the line segments $d_1$, $d_3$. Although the largest possible lengths of the chords $d_2$, $d_4$ are difficult to define because they are variable with their inclination, the arcs of the bows possessing these chords $d_2$, $d_4$ are desirably having a length of not more than 2/3 of the length of the circumference of the blood passing space 202. When the empty space portions 202a, 202b destitute of heat-exchanging tubes 204 are formed within the blood passing space 202, the number of heat-exchanging tubes 204 to be disposed inside the blood passing space 202 naturally decreases proportionaly. When the empty space portions 202a, 202b are given the shape described above, the decrease in the number of heat-exchanging tubes brings about substantially no discernible decline of the efficiency of exchange of heat. Further, when the two empty space portions 202a, 202b destitute of heat-exchanging tubes 204 have a total volume of less than 10% of the volume of the blood passing space 202, the decline of the efficiency of exchange of heat is practically nil.

A heat exchanger comprising a cylindrical blood passing space 202 measuring 55.6 mm in radius and 80 mm in length, a blood inlet tube 205 and a blood outlet tube 206 both measuring 12 mm in inside diameter and disposed as described above and allowed to communicate with the blood passing space 202, and a total of 223 heat-exchanging tubes 204 each measuring 2.14 mm in inside diameter and 2.38 mm in outside diameter and distributed uniformly separated mutually throughout the entire blood passing space 202 except for empty space portions 202a, 202b formed in the aforementioned shape in front of the openings for communication of the blood inlet tube 205 and the blood outlet tube 206 with the blood passing space 202 and occupying a total of 10% of the volume of the blood passing space 202 and another heat exchanger identically constructed, except that the empty space portions 202a, 202b were not formed and heat-exchanging tubes 204 (amounting to 253) were distributed throughout the entire blood passing space 202 including the spaces otherwise occupied by the empty space portions 202a, 202b were produced and actually operated by feeding blood thereto at a rate of 4 liters/minute to test for performance. It was consequently found that the efficiency, $\eta$, of exchange of heat was equally 0.4 for both the heat exchangers and the pressure loss was 1 mm $H_2O$ for the former heat exchanger and 5 mm $H_2O$ for the latter heat exchanger. The results clearly evindence the superiority of performance due to the provision of the empty space portions 202a, 202b destitute of heat-exchanging tubes 204.

The heat exchanger for medical, treatment of the present embodiment which is constructed as described above is put to use as suitably incorporated in a varying extracorporeal circulation path. Since it enjoys exceptional compactness and high performance, it can be integrated with an oxygenator and a blood storing tank and used advantageously in the form of an oxygenator system as illustrated in Fig. 6 and Fig. 7, similarly to the heat exchanger of the embodiment of Fig. 3 to Fig. 5.

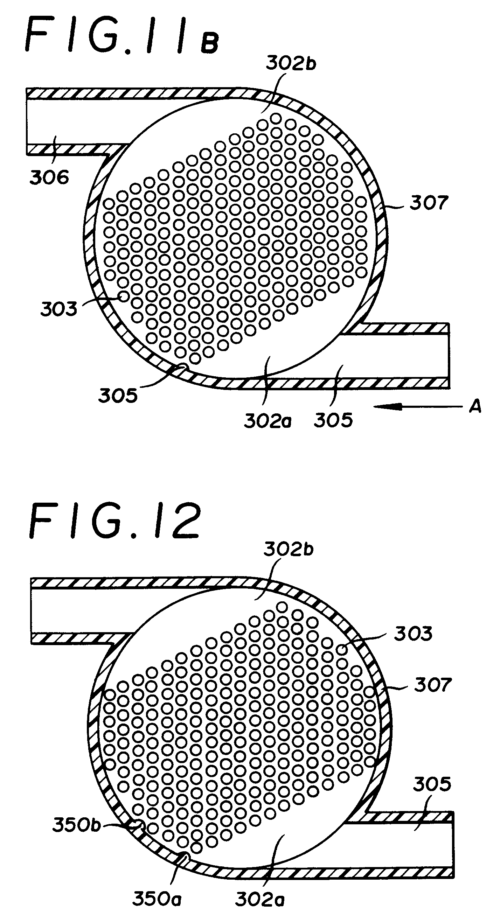

Fig. 11A illustrates yet another embodiment of the present invention; specifically a heat exchanger constructed similarly to that of the embodiment illustrated in Fig. 3 to Fig. 5, except that a housing body 307 is provided on the inner wall surface thereof with a rib 350 disposed parallelly to a slender tube opposed to the inner wall surface so as to retard the flow of blood between the slender tube 303 and the housing body 307.

Fig. 11B illustrates still another embodiment of the present invention, specifically a heat exchanger constructed similarly to that of the embodiment illustrated in Fig. 8 and Fig. 9, except that a housing body 307 is provided on the inner wall surface thereof with a rib 350 disposed parallelly to a slender tube 303 opposed to the inner wall surface so as to retard the flow of blood between the slender tube 303 and the housing body 307 and an empty space portion 302a destitute of slender tube 303 is formed near a blood inlet 305 inside the housing body 307. This empty space may not be formed as in the construction of the heat exchanger of Fig. 11A. When this empty space portion is formed, this portion forms a blood chamber part of low inflow resistance and permits a reduction in pressure loss and the blood entering the housing body 307 is allowed to flow into the whole volume of the empty space portion 302a, form a blood flow extending the entire width of the slender tube 303, and then find its way into the bundle of slender tubes 303. The heat exchanger of this embodiment, therefore, is capable of forming a blood flow extending throughout the entire length of the slender tube 303 and effecting exchange of heat with enhanced efficiency.

The heat exchanger is likewise desirably having an empty space portion 302b destitute of slender tube 303 formed near the position at which a blood outlet 306 is located within the housing body 307. This empty space portion 302b forms a blood chamber part, temporarily accommodates the part of blood which has flowed through the interior of the housing body 307, and releases the blood gradually and, as the result, serves the purpose of precluding the possibility that any part of the blood will form a flow which continuously circulates within the housing body 307 and fails to depart from the housing body 307.

In the heat exchangers illustrated in Fig. 11A and Fig. 11B, the rib 350 formed therein as described above is capable of retarding the flow of blood along the inner wall surface of the housing body 307. The rib 350 is desirably at least extending throughout the entire part of the adjacent slender tube 303 which is exposed to the flowing blood.

Where the slender tubes 303 are accommodated as substantially uniformly separated mutually within the housing body 307, the distance between the rib 350 and the adjacent slender tube 303 is desirably smaller than the distance between the separated slender tubes. This specific distance permits the flow of blood along the inner wall surface of the housing body 307 to be retarded without fail. More desirably, the rib 350 is in substantial contact with the adjacent slender tube 303. The term "substantial contact" as used herein refers to a distance of not more than 0.2 mm, though variable with the pitch between the separated slender tubes. The rib 350 thus disposed relative to the adjacent slender tube 303 is capable of substantially completely eliminating the flow of blood along the inner wall surface of the housing 302.

Desirably, the rib 350 is disposed near the particular slender tube 303 located in the proximity of the blood inlet 305. The provision of just one rib 350 is sufficient for the purpose mentioned above. The heat exchanger may be provided with a plurality of such ribs 350 on condition that they are so shaped and positioned as to avoid retention of bubbles. Particularly where the blood inlet 305 is so attached as to be

13

EP 0 297 970 B1

directed toward the inner surface side of the housing body 307 at a stated angle from the centre of the housing body 307 as illustrated in Fig. 11A and Fig. 11B, since the flow of blood along the inner wall surface of the housing substantially advances in the direction of the blood inlet 305 (the direction indicated by the arrow A), at least one rib 350 disposed on the inner wall surface of the housing toward which the blood inflow 305 is directed is sufficiently capable of retarding the greater part of the flow of blood along the inner wall surface of the housing.

The heat exchanger of the present embodiment has been so far described as having a housing body 307 provided with a heat-exchanging medium inlet and a heat-exchanging medium outlet. This embodiment need not be limited to this construction. The heat exchanger may be modified by disposing partition walls each one in the opposite end parts of the housing body and attaching to the outer surface of one of the partition walls a cap-shaped medium inlet side port provided with heat-exchanging medium inlets communicating with the inner spaces of the slender heat-exchanging tubes and to the outer surface of the other partition wall a cap-shaped medium outlet side port provided with a heat-exchanging medium outlet. The attachment of these ports may be attained by the use of a clamping ring or by means of fusion with ultrasonic waves or high-frequency waves or adhesion with adhesive agent, for example.

While the use of just one rib is sufficient at times, use of two ribs 350a, 350b illustrated in Fig. 12 or use of more ribs proves to be desirable at other times.

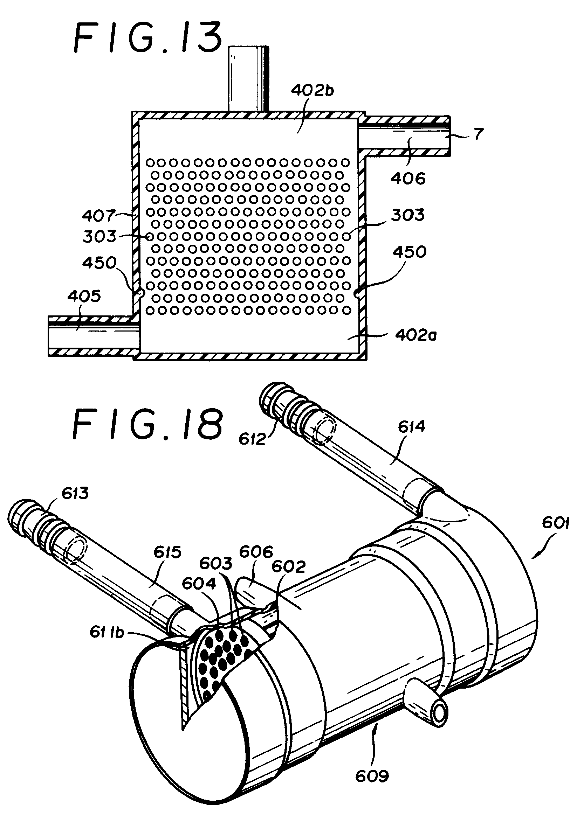

Now, a heat exchanger for blood as a still further embodiment of this invention will be described below with reference to Fig. 13.

The difference between the embodiment illustrated in Fig. 13 and that illustrated in Fig. 11A and Fig. 11B consists in the shape of the housing body 307. In Fig. 13, therefore, the parts which have equivalents in Fig. 11A and Fig. 11B will be denoted by the like reference numerals plus 100.

A housing body 407 is formed in the shape of a polygonal cylinder.

In the embodiment illustrated in Fig. 13, a blood inlet 405 is attached in a direction parallel to one of the surfaces of the polygonal housing body 407. To be more specific, it is so attached as to protrude perpendicularly from the end part of one of the planes of the polygonal housing body 407. A blood outlet 406 is similarly attached in a direction parallel to one of the surfaces of the polygonal housing body 407. To be more specific, it is so attached as to protrude Perpendicularly from the end part of one of the surfaces of the polygonal housing body 407. Desirably, the blood outlet 406 is disposed in a direction substantially Parallel and opposite to the blood inlet 405. The heat exchanger is provided with an empty space portion 402a destitute of slender tubes 403 near the position at which the blood inlet is located in the housing body 407 to ensure effective introduction of blood in spite of the attachment of a blood circuit or an oxygenator to the blood outlet. The empty space portion 402a thus provided in the housing body 407 temporarily accommodates the blood entering the housing and prevents the blood from directly advancing into the bundle of slender tubes. The blood which has filled this empty space portion 402a is then allowed to rise upwardly and then flow inside the housing body 407.

The heat exchanger is further provided with an empty space portion 402b destitute of slender tubes 403 near the position at which the blood outlet 406 is located inside the housing body 407. The empty space portion 402b thus provided in the housing body 407 temporarily accommodates a multiplicity of blood flows passed through the inner spaces of the bundle of slender tubes and combines them into one blood flow destined to advance toward the blood inlet 406. Owing to this function of the empty space portion 402b, the multiplicity of blood flows about to rise through the inner spaces of the bundle of slender tubes are allowed to continue their advance through the bundle of slender tubes without being affected by an external force tending to speed up or slow down the flow.

The slender tubes 403 are arranged within the housing body 407 in rows parallel to the direction of the blood inlet as illustrated in Fig. 13. The individual rows of slender tubes are so disposed that the individual slender tubes in the adjacent rows are staggered. In this arrangement, therefore, the blood flows rising from below find their way through the spaces intervening between the individual slender tubes in the lower row, collide against the individual slender tubes of the immediately upper row, then find their way through the spaces intervening between the same individual slender tubes, and continue their ascent by repeating these motions through the rest of the rows of slender tubes. The rows thus formed of the slender tubes 403 may be slanted at a stated angle from the direction of the blood inlet instead of being perfectly parallel thereto.

The housing body 407 is provided on the inner wall surface thereof with a rib 450 which is extended in the axial direction of the housing body 407 parallelly to a particular slender tube 403 opposed to the portion of the inner wall surface of the housing destined to seat the rib, so as to retard the flow of blood between the adjacent slender tube 403 an the inner wall surface of the housing body 407. The rib 450 disposed as described above in the housing body 407 is capable of dispersing the flow of blood along the inner wall surface of the housing body 407. The rib 450 is desired at least to be extended throughout the entire part of

14

EP 0 297 970 B1

the adjacent slender tube 403 which is exposed to contact with the blood.

Where the slender tubes 403 are accommodated substantially uniformly separated mutually within the housing body 407, the distance between the rib 450 and the adjacent slender tube 403 is desired to be smaller than the distance between the separated slender tubes. This specific distance permits the flow of blood along the inner wall surface of the housing body 407 to be retarded without fail. More desirably, the rib 450 is in substantial contact with the adjacent slender tube 403. The term "substantial contact" as used herein refers to a distance of not more than 0.2 mm, though variable with the pitch between the separated slender tubes. The rib 450 thus disposed relative to the adjacent slender tube 403 is capable of substantially completely eliminating the flow of blood along the inner wall surface of the housing 402.

Desirably, this rib 450 is disposed near the particular slender tube 403 which is located near the blood inlet 405. Further in the present embodiment, the blood which has filled the empty space portion is allowed to ascend and continue its flow inside the housing. Similarly to the embodiment illustrated in Fig. 6, the blood flows more readily between the inner wall surface of the housing body 407 and the slender tubes opposed to the inner wall surface than through the spaces between the individual slender tubes in the present embodiment. Further in the present embodiment, since no appreciable difference occurs in the flow rate of blood between the opposed surfaces of the polygonal housing body, ribs may be formed on each one of the opposed inner surfaces of the housing.

The opposite end parts of the slender tube 403 are watertightly fixed to the housing body 407 with partition walls similarly to those in the embodiment of Fig. 6. A heat-exchanging medium inlet is formed near the end part of the housing beyond one of the partition walls and a heat-exchanging medium outlet near the end part of the housing beyond the other partition wall. The heat-exchanging medium inlet and the outlet both communicate with the inner spaces of the slender het-exchanging tubes. A seal member is fitted in one end part of the housing. Another seal member is similarly fitted in the other end part of the housing.

The housing, instead of being provided with the heat-exchanging medium inlet and the heat-exchanging medium outlet, may be provided with partition walls disposed each one in the end parts of the housing, a cap-shaped medium inlet side port possessed of a heat-exchanging medium inlet communicating with the inner spaces of the slender heat-exchanging tubes and formed in the outer surface of one of the partition walls , and cap-shaped medium outlet side port possessed of a heat-exchanging medium outlet and formed in the outer surface of the other partition wall. The fixation of these ports may be effected by the use of clamping rings or by means of fusion with ultrasonic waves or highfrequency waves or adhesion with adhesive agent.

As slender heat-exchanging tubes, the heat exchanger of the present invention is desirably provided in the housing thereof with about 10 to 2,000, preferably about 50 to 1,000, metallic tubes of high thermal conductivity (such as, for example, stainless steel tubes, aluminum tubes, or copper tubes) having an inside diameter in the range of 0.5 to 10 mm, preferably 2 to 5 mm. These slender tubes are accommodated in the housing parallelly to the axial direction of the housing. The blood introduced through the blood inlet, therefore, flows inside the housing in such a direction as to traverse the slender tubes. The slender tubes are separated by a fixed distance. This distance, though variable with the outside diameter of slender tube or the inside diameter of housing, for example, generally falls approximately in the range of 0.2 to 4 mm, preferably 0.8 to 2 mm.

The housing is made of any of various materials such as polycarbonate, acryl-styrene copolymer, and acryl-butylene-styrene copolymer. The seal members used for sealing the opposite ends of the housing are discs possessed of an outer contour substantially equal to the inner contour of the end parts of the housing and made of any of various materials such as polycarbonate, acryl-styrene copolymer, and acryl-butylene-styrene copolymer. These seal members are fixed watertightly to the end part of the housing by means of adhesion with adhesive agent or solvent or fusion with high-frequency waves, ultrasonic waves, or induction heating.

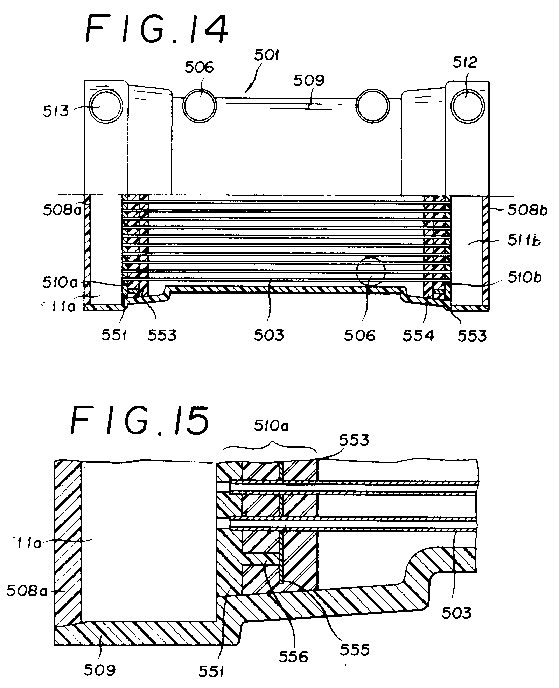

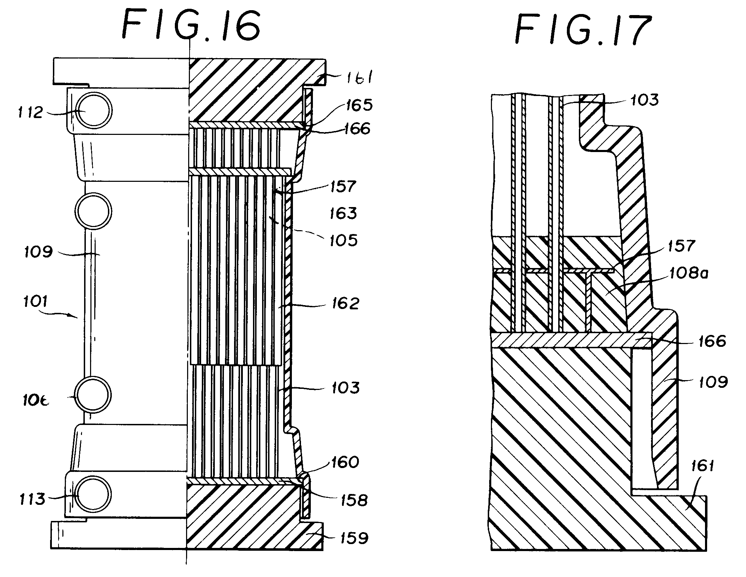

Fig. 14 and Fig. 15 illustrate a further embodiment of the present invention. The difference between the heat exchanger of this embodiment and that of the embodiment illustrated in Fig. 3 to Fig. 5 consists mainly in the construction of partition walls 510a, 510b. In Fig. 14 and Fig. 15, the parts which have equivalents in Fig. 3 to Fig. 5 are denoted by like reference numerals plus 400. Fig. 15 is a magnified cross section illustrating a partition wall and adjacent parts of the heat exchanger of Fig. 14.

A cylindrical housing 509 of a heat exchanger 501 accommodate therein a multiplicity of slender heat-exchanging tubes 503. The housing 509 and the slender tubes 503 are similar to those already described. In the present embodiment, partition walls 510a, 510b are formed respectively of perforated plates 551, 552 and potting compounds 553, 554. The partition walls will be described specifically with reference to Fig. 15. The perforated plate 551 possesses a multiplicity of holes each having an inside diameter larger than the outside diameter of slender tube 503 at one end thereof and an inside diameter smaller than the outside

15

diameter of slender tube 503 at the other end thereof. In the embodiment of Fig. 15, the perforated plate 551 possesses holes which have an inside diameter converged from one end to the other thereof so as to decrease past the outside diameter of the slender tubes 503 halfway along the wall thickness of the perforated plate. The end parts of the slender tubes 503 are inserted into these holes in the perforated plate. Further, the perforated plate 551 is provided with a plurality (at least two)ribs 556 serving the purpose of keeping a slender tube dispersing plate 555 apart from the perforated plate 551. The slender tube dispersing plate 555 is intended to impart a fixed pattern to the bundle of slender tubes 503 in a dispersed state and, therefore, is provided with a multiplicity of holes fit for insertion of the slender tubes 503. The provision of the slender tube dispersion plate and the ribs, though not an indispensable requirement, proves to be desirable in the sense that it ensures uniform dispersion of the slender tubes 503. The potting compound 553 fixes the slender tubes 503 watertightly to the perforated plate 551 and the perforated plate 551 similarly watertightly to the housing 509. The slender tube dispersing plate 555 is completely buried in the potting compound. The complete embedment in the potting compound brings about an advantageous effect in preventing the blood under treatment from coming into direct contact with the dispersing plate. The partition walls 510b and the adjacent parts are similarly constructed.

Now, the method employed for the production of the heat exchanger for medical tretment of the present invention illustrated in Fig. 3 to Fig. 5 will be described below with reference Fig. 16. Fig. 16 is a process diagram illustrating the flow of the steps in the method for the production of the heat exchanger of this invention.

The method for the production contemplated by the invention comprises the steps of forming a cylindrical housing possessing heat-exchanging medium ports 112, 113 disposed one each at the opposite end parts thereof and a blood inlet port 105 and a blood outlet port 106 disposed at positions between the medium ports 112, 113, attaching a first sealing member at a position between the medium port (medium inlet port 113, for example) and the blood port (blood outlet port 106, for example) located at one end part of the cylindrical housing 109, inserting through the other end part of the cylindrical housing 109 into the cylindrical housing a slender tube distributing device 158 possessing a multiplicity of holes fit for insertion of slender heat-exchanging tubes and a multiplicity of slender heat-exchanging tubes 103, attaching a second sealing member at a position between the medium port (medium outlet port 113, for example) and the blood port (blood inlet port 105 or blood outlet port 106, for example) which are located at the other end part of the cylindrical housing 109, injecting a potting compound through the blood port (blood outlet port 106, for example) on one end part side and the blood port (blood inlet port 105 or blood outlet port 106, for example) on the other end part side of the cylindrical housing 109 with the end parts of the slender heat-exchanging tubes kept in a closed state, thereby forming partition walls 110a, 110b for fixing the opposite end parts of the slender heat-exchanging tubes 103 to the cylindrical housing 109, removing the first sealing member and the second sealing member, and fitting seal membrs 111a, 111b one each to the opposite ends of the cylindrical housing 109. To be more specific, after the housing 109 has been formed, one end part of the housing 109 is closed fast by having the sealing member fitted to the end part of the housing 109 at a position (falling between the medium inlet port 113 and the blood outlet port 106, for example) approximating the medium port. The first sealing member is intended to close the end parts of the slender heat-exchanging tubes 103 when the second sealing member is depressed into place as described later on. The sealing members are desired to be formed of an elastic sealing member 158 (a rubber sheet of silicone rubber, polyurethane rubber, or latex rubber, for example) possessing a cross-sectional contour matched to that of the housing 109 as held in the aforementioned place and a retaining device 159 serving to keep the elastic sealing member in place. The elastic sealing member 158 must be incapable of adhering to the potting compound which is poured into the housing 109 later. This requirement may be met by using the potting compound and the elastic sealing member which are made of materials devoid of adhesion quality (silicone rubber sheet for the elastic sealing member and polyurethane as the potting compound or silicone rubber as the potting compound and polyurethane for the elastic sealing member, for example) or, where the materials therefor both possess adhesive quality, by coating the surface of the elastic sealing member 158 falling on the inner surface side of the housing 109 with resin of the kind capable of cancelling the adhesive quality of the materials (such oil as silicone oil for example). The end parts of the housing 109, as illustrated in Fig. 5 and Fig. 16, are radially diverged in the direction of their extremities from the positions approximating the medium ports 112, 113. The end part of the housing 109 is closed by having the elastic sealing member 158 fitted into the radially diverged portion 160. The possible separation of the elastic sealing member 158 from the housing is precluded by the retaining device 159.

Subsequently, the housing 109 is held upright on the side thereof provided with the aforementioned retaining device 159 and the slender tube-dispersing device 157 is inserted in the housing 109. The slender tube-dispersing device is intended to impart a state pattern to the bundle of slender tubes 103 to be held in

EP 0 297 970 B1

a mutually separated state and, therefore, provided with a multiplicity of holes for insertion of the slender tubes 103. In the embodiment illustrated in Fig. 15, the slender tube-dispersing device 157 comprises a plate 161 possessing multiplicity of holes matched to the slender tubes 103 in a bundled state and a multiplicity of pipes 162 having the end parts thereof inserted in the holes of the plate 161. The pipes 162 are fixed in the plate 161. The pipes 162 have an inside diameter so larger than the outside diameter of the slender tubes 103 that the slender tubes 103 can be inserted into the pipes 162. The largest part of the plate 161 has an outside diameter larger than the inside diameter of the middle part of the housing 109 and smaller than the radially diverged portion 163 so that when this plate is inserted into the housing 109, it will be hung down from the radially diverged portion 163 as illustrated in Fig. 16. The pipes 162 have a length so adjusted that they will not reach the blood outlet port 106 located below when the slender tube-dispersing device 157 is inserted into the housing 109 (namely hung down from the radially diverged portion 163 with the plate 161). This adjustment of length is necessary for the purpose of preventing the pipes 162 of the slender tube-dispersing device 157 from being fixed with the potting compound which will be poured in through the blood outlet port 106 later on. The slender tube-dispersing device using the pipes in the manner described above permits the bundle of slender tubes to be dispersed certainly in a desired pattern. Further, owing to the use of such pipes as mentioned above, the slender tubes 103 and the slender tube-dispersing device 157 contact each other in large portions enough for the slender tubes to be steadily retained in a dispersed state and to be prevented from otherwise possible disruption of the dispersed state under the impact of the potting compound during the injection of the potting compound. After the insertion of the slender tube-dispersing device 157, the slender tubes 103 are inserted into the pipes 162 which has been already inserted through the holes of the plate 161 of the dispersing device 157. The slender tubes 103 thus inserted have the lower end parts thereof thrust out of the leading ends of the pipes 162., The leading ends of the slender tubes 103 come into contact with the elastic sealing member 158. The slender tubes 103 have a length so adjusted that the upper ends thereof fall in the neighborhood of the radially diverged portion of the housing 109. Then, the second sealing member is fitted downwardly into the radially diverged portion 165 of the housing 109 to seal the other end part of the housing 109. The second sealing member, similarly to the first sealing member, is desirably comprising an elastic sealing member 166 possessing a cross-sectional contour matched to that of the housing 109 and is held in the place mentioned above and a retaining device 161 adapted to keep the elastic sealing member 166 in place. This sealing member is advantageously made of the same material as mentioned above. When the retaining device 161 is depressed into place from above, the end parts of the slender tubes 103 are closed with the elastic sealing members 158, 166. The heat exchanger has been so far described as being provided with the elastic sealing member 166. Since it is only required to close the end parts of the slender heat-exchanging tubes, the installation thereof is not necessarily indispensable up to this point in the whole course of the production. Then, the potting compound is poured in through the blood outlet port 106 and allowed to set. Subsequently, the upper retaining device 161, the elastic sealing member 166, and the slender tube-dispersing device 157 are removed. Further, the elastic sealing member 160 is fitted in the radially diverged portion 165 of the housing 109 to close the other end of the housing 109 and set in place with the retaining device 161. Now, the housing 109 is turned upside down and the retaining device 159 is depressed into place from above to close the end parts of the slender tubes 109. In the same manner as described above, the potting compound is poured in through the blood outlet port (or the blood inlet port 105) and allowed to set. After the potting compound has been solidified, the retaining devices 159, 161 and the elastic sealing members 158, 166 attached to the opposite end parts of the housing 109 are removed. Consequently, the partition walls 110a, 110b are formed.

Since the method described above requires the removal of the slender tube-dispersing device as one of the essential steps thereof, the injection of the potting compound can be attained infallibly and easily even on the side used for the insertion of the slender tube-dispersing device 157. Since the opposite ends of the slender tubes 103 are kept in place with the elastic sealing members 158, 166, the elastic sealing members absorb any possible minor variation of length of the slender tubes 103 and are capable of preventing the potting compound from flowing into the slender tubes 103. Further, the seal members 108a, 108b possessing a cross-sectional contour matched to that of the opposite ends of the housing 109 are fixed watertightly to the opposite end parts of the housing 109. The fixation of the seal members is attained by adhesion with adhesive agent or by fusion with high-frequency waves, ultrasonic waves, or induction heating.

The heat exchanger has been so far described as being provided with sealing members comprising elastic sealing members 158, 166 and retaining devices 159, 161. Optionally, the sealing members used for the heat exchanger may be of the type produced by integrally forming the elastic sealing members and the retaining devices.

17

The insertion into the cylindrical housing 109 through the other end part of the cylindrical housing 109 of the slender tube-dispersing device possessing multiplicity of holes of insertion of slender heat-exchanging tubes and the multiplicity of slender heat-exchanging tubes may be carried out with the housing kept in a horizontal state. The insertion carried out with the housing kept in an upright state proves to be more desirable because the slender tubes can be inserted with greater ease. The insertion into the cylindrical housing 109 through the other end part of the cylindrical housing 109 of the slender tube-dispersing device possessing multiplicity of holes for insertion of slender heat-exchanging tubes and the multiplicity of slender heat-exchanging tubes may be effected, for example, by first inserting the slender tubes 103 through the holes of the slender tube-dispersing device 157 and then causing the slender tube-dispersing device 157 now holding the slender tubes 103 inserted therethrough to be inserted in the housing 109. The formation of the partition walls may be carried out, for example, by rotating the housing 109 about the axis thereof while keeping the end parts of the slender heat-exchanging tubes 103 fast in place, then centrifugally pouring the potting compound through the blood port on one end part side of the cylindrical housing 109, allowing the introduced potting compound to set, and repeating the same procedure on the other end side. Otherwise, it may be effected by rotating the housing about the central part of the housing and, at the same time, centrifugally pouring the potting compound simultaneously through the blood ports on the opposite end parts and allowing the introduced potting compound to set.

The step of fitting the seal members 508a, 508b, to the opposite ends of the housing 109 may comprise first removing either the first sealing member or the second sealing member, fitting one of the seal members to the end part on the side on which the removal just mentioned has been made, removing the remaining sealing member, and fitting the other seal member to the end part on the side on which the removal has been made.

The method for production has been so far described as requiring the removal of the slender tube-dispersing device as one of the component steps. When the slender tube-dispersing device 157 is such that the continued presence thereof within the housing offers no hindrance whatever to the use of the heat exchanger, it need not be removed by all means. As a concrete example of the slender tube-dispersing device 157 which answers the description, a plate formed of a metallic substance such as stainless steel or a synthetic resin as illustrated in Fig. 16 may be cited. This slender tube-dispersing device 157 is provided with a plate part possessing a multiplicity of holes permitting insertion therein of the slender tubes 103 and a plurality (at least three) of leg parts extended downwardly from the plate part. The plate part has a lateral part thereof cut away to permit downward flow of the potting compound. When the slender tube-dispersing device of this construction is used in pouring the potting compound through the blood port, it is eventually buried in the partition wall to be formed of the potting compound. Thus, this slender tube-dispersing device places no hindrance whatever in the way of actual use of the heat exchanger.

Fig. 18 is a partially cutaway perspective view illustrating typical heat exchanger for medical treatment as still another embodiment of this invention. As clearly noted from the partition wall, a heat exchanger 601 of this embodiment similar to the heat exchanger of the embodiment illustrated in Fig. 3 to Fig. 5 is provided in one heat-exchanging medium passing space with a heat-exchanging medium outlet port 612 and in the other heat-exchanging medium passing space 611b with a heat-exchanging medium inlet port 613.

In the heat exchanger 601 of the present embodiment, the heat-exchanging medium outlet port 612 and the heat-exchanging medium inlet port 613 are not formed integrally with a housing 609 but are formed by being extended from the outer all surfaces of the housing 609 with flexible tubes 614, 615. The flexible tubes 614, 615 are only required to exhibit substantial and ample flexibility and permit suitable change of positions of the heat-exchanging medium outlet port 612 and the heat-exchanging medium inlet port 613 attached to the leading ends of the flexible tubes 614, 615 and, further, desirably to enable the flexible tubes themselves to be effectively pinched with clamps. They are not discriminated particularly by such factors as material, length, and wall thickness.

Further in the embodiment illustrated in Fig. 18, the second fluid which flows in the closed space of the housing is a heat-exchanging medium. To be specific, this heat exchanger is so constructed that the heat-exchanging medium is passed outside the heat-exchanging tubes 604. The heat exchanger for medical treatment in the present embodiment may be alternatively constructed so that the heat-exchanging medium will flow through the inner spaces of the heat-exchanging tubes 604 (namely the first fluid passing through the inner spaces of the heat-exchanging tubes will be the heat-exchanging medium).

The heat exchanger 601 for medical treatment of the present embodiment which is constructed as described above possesses a highly desirable operating property and is used advantageously as incorporated in a varying kind of extracorporeal circulation path. For example, it can be used advantageously as integrated with an oxygenator and a blood storing tank as illustrated in Fig. 6 and Fig. 7 and allowed to function as an oxygenator system.

In the oxygenator system to be formed by integrating the heat exchanger for medical treatment with the blood storing tank and the oxygenator as described above, the blood withdrawn from the patient's body first flows into the heat exchanger 601 through the blood inlet tube. In the meantime, the water introduced as a heat-exchanging medium through the heat-exchanging medium inlet port 613 connected by means of a coupler, for example, to a connection tube (not shown) led out of the heat-exchanging medium temperature controller is passed into the heat-exchanging medium passing space, forwarded through the inner spaces 603 of the heat-exchanging tubes 604, brought to the heat-exchanging medium passing space 611b, discharged from the heat-exchanging medium outlet port 612 into another connection tube (not shown) led out of the heat-exchanging medium temperature controller, and returned to the heat-exchanging medium temperature controller. The exchange of heat across the walls of the heat-exchanging tubes 604 within the blood passing space 602 of this heat exchanger 601 for medical treatment is effected efficiently and uniformly. The blood which has been heated or cooled to a desired temperature in consequence of the exchange of heat is led out of the heat exchanger 601 through the blood outlet tube 606 and then forwarded to the oxygenator 121 through the blood inlet tube 131 of the oxygenator 121 communicating watertightly with the blood outlet tube 606. The blood introduced through the blood inlet tube 131 of the oxygenator 121, while passing through the blood chamber 130, exchanges gas with the oxygen-containing gas flowing through the inner spaces of the hollow fiber membranes 124 through the medium of the hollow fiber membrances 124. As the result, the blood is divested of excessive carbon dioxide gas and replenished with fresh oxygen. The blood which has been oxygenated flows out of the blood outlet tube 132 of the oxygenator 121 and advances into the blood storing tank 141 through the blood inlet 142 of the blood storing tank 141. The blood which has flowed from the blood inlet 142 to the communicating blood inlet part 143 and then reached the deforming member 147 disposed en route to the blood inlet part 143 is defoamed during the passage thereof through the defoaming member 147 by the fact that the multiplicity of bubbles contained in the blood come into the foam cells of the defoaming member 147, grow by agglomeration, and pass from within the blood into the upper empty space within the blood storing tank 141. The defoamed blood moves on to the communicating blood storing part 144, remains temporarily in the blood storing part 144, departs from the blood outlet 145 disposed below the blood storing part 144, and returns to the patient's body. In the heat exchanger 601 for medical treatment in the present embodiment, the heat-exchanging medium inlet port 613 and the heat-exchanging medium outlet port 612 are disposed as extended with the flexible tubes 614, 615 from the outer wall surfaces of the housing 609 as described above. If a variation occurs in the positional relation between the heat exchanger 601 for medical treatment and the heat-exchanging medium temperature controller during the course of the extracorporeal circulation, it can be completely absorbed by the freedom of twist enjoyed by the flexible tubes 614, 615. Thus, the possibility of their disconnection during the course of the operation mentioned above is nil.

After the extracorporeal circulation treatment is completed, the blood remaining in the oxygenator system must be recovered. This recovery is easily obtained by clamping the flexible tubes 614, 615 of the heat exchanger 601 for medical treatment because the oxygenator system is now ready to be tilted freely without entailing the otherwise possible leakage of the heat-exchanging medium.

Example 1

A cylindrical housing was used which was formed of polycarbonate in the shape (72 mm in inside diameter, 79 mm in outside diameter, and 150 mm in length) illustrated in Fig. 3 and Fig. 11B, with a blood inlet 105 disposed on the lower part of the housing and two blood outlets on the upper part of the housing, the blood inlet and the blood outlets attached to the housing in directions substantially tangent to the outer surface of the housing, and heat-exchanging medium inlet and outlet disposed near the end parts of the housing.

As slender heat-exchanging tubes, 223 of stainless steel tubes (2.14 mm in inside diameter) were accommodated inside the housing as mutually separated by a distance of 0.82 mm. Inside the housing, empty space portions destitute of slender tube were formed each one near the positions at which the blood inlet and the blood outlets were located.

On the inner wall surface of the housing, one rib 350a of the shape illustrated in Fig. 13 was disposed as extended in the axial direction of the housing parallelly to the particular slender tube opposed to the inner wall surface of the housing seating the rib. This rib measured 3 mm in width and 1.5 mm in height. The distance between the rib and the adjacent tube was 0.4 mm. Since the housing was radially diverged toward the end parts thereof, this distance was found as an average of distances measured in the end parts and in the central part. The slender tubes were fixed to the housing by using polyurethane as a potting compound. A heat exchanger contemplated by this invention was completed by closing the opposite end parts with seal members.

Example 2

A heat exchanger was produced by following the procedure of Example 1, except that a housing was formed of polycarbonate in the shape illustrated in Fig. 3 and Fig. 11A and one rib 350 was disposed on the inner wall surface of the housing.

Example 3

A heat exchanger was produced by following the procedure of Example 1, except that a rib was disposed at the position indicated 350b in Fig. 13.

Example 4

A heat exchanger was produced by following the procedure of Example 1, except that no rib was formed on the inner wall surface of the housing.

Example 5

A heat exchanger was produced by following the procedure of Example 2, except that no rib was formed on the inner wall surface of the housing.

Example 6

The heat exchangers obtained in Examples 1 to 5 were each subjected to the following test. In the test, water was used in the place of blood and water was also used as a heat-exchanging medium.

To a given heat exchanger, water at a temperature of 40°C was introduced through the heat-exchanging medium inlet at a rate of 15 liters/min and water at a temperature of 30°C was introduced through the blood inlet at a varying rate of 2 liters, 4 liters, or 6 liters per minute. The temperature of the water at the blood inlet (TBo) and the temperatue of the water at the medium inlet (TWo) were measured.

The efficiency of exchange of heat ($\eta$) was calculated from the following formula using the results of measurement:

$$\eta = (TBo - TBi)/(Twi - TBi)$$

wherein TBi stands for the temperature of the water flowing in through the blood inlet, and TWi for the temperature of the water flowing in through the heat-exchanging medium inlet. The results of the test are shown in Table 1.

Table 1

| Flow volume(liters) | Efficiency of exchange of heat($\eta$) | | | | |
|---|---|---|---|---|---|
| | Example | | | | |
| | 1 | 2 | 3 | 4 | 5 |
| 2 | 0.76 | 0.75 | 0.74 | 0.64 | 0.63 |
| 4 | 0.57 | 0.57 | 0.56 | 0.49 | 0.50 |
| 6 | 0.45 | 0.46 | 0.46 | 0.41 | 0.43 |

The heat exchanger for medical treatment of this invention comprises a cylindrical blood passing space and a multiplicity of heat-exchanging tubes disposed inside the blood passing space in the longitudinal direction of the blood passing space and provided with an inner space watertightly separated from the blood passing space and effecting exchange of heat between the blood being passed through the blood passing space and a heat-exchanging medium being passed through the inner spaces of the heat-

EP 0 297 970 B1

exchanging tubes through the medium of walls of the heat-exchanging tubes and this heat exchanger is characterized by the fact that a blood inlet tube for introducing blood into the blood passing space and a blood outlet tube for discharging blood from within the blood passing space are severally extended inwardly from outside substantially along straight lines perpendicular to the longitudinal direction of the blood passing space and tangent to the peripheral plane of the blood passing space and allowed to communicate with the blood passing space as described above. It is, therefore, capable of causing the blood flowing through the blood passing space to be uniformly distributed within the blood passing space, effecting uniform exchange of heat with the entire blood passed through the heat exchanger, and exhibiting stable performance without exerting any adverse effect upon the blood components even when the temperature distribution of the blood is dispersed and the exchange of heat consequently occurs excessively or insufficiently.

The heat exchanger for medical treatment of the present invention is produced by causing a multiplicity of heat-exchanging tubes to be disposed as mutually separated inside a cylindrical housing possessing closed opposite ends in the longitudinal direction of the housing, partition walls disposed at the opposite end parts of the heat-exchanging tubes to hold the heat-exchanging tubes fast watertightly on the lateral wall of the housing without closing the openings of the heat-exchanging tubes and, at the same time, partition the interior of the housing into three spaces, a blood inlet tube and a blood outlet tube severally extended inwardly from outside substantially along stright lines perpendicular to the longitudinal direction of the housing and tangent to the peripheral surface of the housing to communicate with a blood passing space formd in the central part of the housing by the two partition walls, the lateral wall of the housing, and the outer walls of the heat-exchanging tubes, and a heat-exchanging medium inlet tube to communicate with one of two heat-exchanging medium passing spaces formed at the end parts of the housing communicating with the inner spaces of the heat-exchanging tubes watertightly separated from the blood passing space and a heat-exchanging medium outlet tube to communicate with the other heat-exchanging medium passing space. It enjoys compactness of construction and high performance and, therefore, can be advantageously used as integrated with an oxygenator, for example. It is capable to effect the exchange of heat with improved efficiency when the blood inlet tube communicates with the blood passing space near one of the partition walls and the blood outlet tube communicates with the blood passing space near the other partition wall and further the blood inlet tube and the blood outlet tube assume a positional relation such that they are mounted from each other at an angle of about 180° around the peripheral surface of the blood passing space.

The heat exchanger for medical treatment of this invention comprises a cylindrical blood passing space and a multiplicity of heat-exchanging tubes disposed inside the blood passing space in the longitudinal direction of the blood passing space and provided with an inner space watertightly separated from the blood passing space and effecting exchange of heat between the blood being passed through the blood passing space and a heat-exchanging medium being passed through the inner spaces of the heat-exchanging tubes through the medium of walls of the heat-exchanging tubes and this heat exchanger is characterized by the fact that a blood inlet tube for introducing blood into the blood passing space and a blood outlet tube for discharging blood from the blood passing space are severally extended inwardly from outside substantially along straight lines perpendicular to the longitudinal direction of the blood passing space and tangent to the peripheral plane of the blood passing space and allowed to communicate with the blood passing space and said heat-exchanging tubes are disposed as uniformly separated mutually throughout the entire blood passing space except for an empty space portion formed by extending in the axial direction of the blood passing space a portion enclosed in the shape of a bow possessing a chord substantially parallel to and equal to or slightly longer than a line segment connecting two points of intersection between two inner peripheral lines of the blood inlet tube and the circumference of the blood passing space in a cross section perpendicular to the axis of the blood passing space inclusive of the axial line of the blood inlet tube and an empty space-portion formed by extending in the axial direction of the blood passing space a portion enclosed in the shape of a bow possessing a chord substantially parallel to and equal to or slightly longer than a line segment connecting two points of intersection between two inner peripheral lines of the blood outlet tube and the circumference of the blood passing space in a cross section perpendicular to the axis of the blood passing space inclusive of the axial line of the blood outlet tube. It enjoys compactness of construction and high performance and, therefore, can be advantageously used as integrated with an oxygenator, for example. It is capable to effect the exchange of heat with improved efficiency and capable to curb the pressure loss during the course of blood passage when the blood inlet tube communicates with the blood passing space near one of the partition walls and the blood outlet tube communicates with the blood passing space near the other partition wall and further the blood inlet tube and the blood outlet tube assume a positional relation such that they are mounted at an angle of about 180° from each other around the peripheral surface of the blood passing space.

21

The heat exchanger for medical treatment of this invention is produced by causing a multiplicity of heat-exchanging tubes to be disposed as mutually separated inside a cylindrical housing possessing closed opposite ends in the longitudinal direction of the housing, partition wall disposed at the opposite end parts of the heat-exchanging tubes to hold the heat-exchanging tubes fast watertightly on the lateral wall of the housing without closing the openings of the heat-exchanging tubes and, at the same time, partition the interior of the housing into three spaces, a blood inlet tube and a blood outlet tube to communicate with a blood passing space formed in the central part of the housing by the two partition walls, the lateral wall of the housing, and the outer walls of the heat-exchanging tubes, and a heat-exchanging medium inlet tube to communicate with one of two heat-exchanging medium passing spaces formed at the end parts of the housing communicating with the inner spaces of the heat-exchanging tubes watertightly separated from the blood passing space and a heat-exchanging medium outlet tube to communicate with the other heat-exchanging medium passing space. It enjoys compactness of construction and high performance and, therefore, can be advantageously used as integrated with an oxygenator, for example. It is capable to effect the exchange of heat with improved efficiency when the blood inlet tube communicates with the blood passing space near one of the partition walls and the blood outlet tube communicates with the blood passing space near the other partition wall and further the blood inlet tube and blood outlet tube assume a positional relation such that they are mounted at an angle of about 180° from each other around the peripheral surface of the blood passing space and the two empty space portions destitute of heat-exchanging tubes in the blood passing space occupy a total volume of less than 40% of the volume of the blood passing space.

The heat exchanger of this invention comprises a cylindrical housing possessing a blood inlet and a blood outlet, a multiplicity of slender heat-exchanging tubes accommodated within the cylindrical housing, partition walls adapted to fix the opposite end parts of the slender heat-exchanging tubes watertightly to the cylindrical housing and partition the interior of the housing into a blood chamber communicating with the blood outlet and the blood inlet and a heat-exchanging medium chamber formed by the tube interiors, and a medium inlet and a medium outlet communicating with the heat-exchanging medium chamber and this heat exchanger further comprises a rib formed on the inner wall surface of the housing as extended parallelly to the slender tubes opposed to the inner wall surface and adapted to retard the flow of blood between the slender tubes and the inner wall surface of the housing. Particularly since the rib is formed on the inner wall surface of the housing, the blood flowing along the inner wall surface of the housing collides against the rib and then advances in the direction of the spaces intervening between the bundle of slender tubes. Thus, the blood flowing along the inner wall surface of the housing and reaching the blood outlet without undergoing sufficient exchange of heat can be decreased and the efficiency of exchange of heat can be improved.

The heat exchanger of this invention comprises an integrally molded cylindrical housing possessing a heat-exchanging medium inlet port disposed at one end part thereof, a heat-exchanging medium outlet port disposed at the other end part thereof, and a blood inlet port and a blood outlet port disposed at positions between the medium inlet port and the medium outlet port, a multiplicity of slender heat-exchanging tubes accommodated within the housing, partition walls adapted to fix the opposite end parts of the slender tubes watertightly to the cylindrical housing and partition the interior of the housing into a blood chamber communicating with the blood outlet port and the blood inlet port and a heat-exchanging medium chamber formed inside the tubes and adapted to communicate with the medium inlet port and the medium outlet port, and seal members serving to seal the opposite end parts of the housing. Since the heat-exchanging medium inlet port and outlet port are integrally formed with the housing, these inlet port and outlet port can be present at desired positions selected in the housing for each attachment of circuits, for example, during the formation of the housing and the partition walls for watertightly fixing the slender tubes in the housing can be fastened watertightly to the housing infallibly and the otherwise possible leakage of the heat-exchanging medium between the partition walls and the housing can be precluded.

The method for the production of the heat exchanger of this invention comprises the steps of forming a cylindrical housing possessing heat-exchanging medium ports disposed one each at the opposite end parts thereof and a blood inlet port and a blood outlet port disposed at positions between the medium ports, attaching a first sealing member at a position between those of the medium ports and the blood ports which are located at one end part of the cylindrical housing, inserting through the other end part of the cylindrical housing into the cylindrical housing a slender tube distributing device possessing a multiplicity of holes for insertion of slender heat-exchanging tubes and a multiplicity of slender heat-exchanging tubes, attaching a second sealing member at a position between those of the medium ports and the blood ports which are located at the other end part of the cylindrical housing, injecting a potting compound through the blood port on one end part side and the blood port on the other end part side of the cylindrical housing with the end

22

EP 0 297 970 B1

parts of the slender heat-exchanging tubes kept in a closed state thereby forming partition walls for fixing the opposite end parts of the slender heat-exchanging tubes to the cylindrical housing, removing the first sealing member and the second sealing member, and fitting seal members one each to the opposite ends of the cylindrical housing. By this method, the heat exchanger of this invention described above can be easily produced.

The heat exchanger for medical treatment of the present invention comprises a housing for enclosing a closed empty space therewith and heat-exchanging tubes disposed inside the housing and provided each with an inner space watertightly separated from the closed empty space and effecting exchange of heat between a first fluid passed through the inner spaces of the heat-exchanging tubes and a second fluid passed through the closed empty space of the housing through the medium of walls of the heat-exchanging tubes and this heat exchanger is characterized by the fact that a heat-exchanging medium inlet port and a heat-exchanging medium outlet port for causing the first fluid or the second fluid intended as a heat-exchanging medium to be respectively introduced into and discharged from the inner spaces of the heat-exchanging tubes disposed inside the housing or the closed empty space of the housing are extended from the outer wall surface of the housing with flexible tubes. When the heat-exchanging medium inlet port and the heat-exchanging medium outlet port are connected to the connection tube, for example, of the heat-exchanging medium temperature controller, this connection can be easily attained because the heat exchanger and the heat-exchanging medium temperature controller are given a generous allowance with respect to their relative layout. If the positional relation between the heat exchanger and the heat-exchanging medium temperature controller is varied during the course of operation, there is no possibility of their connection being dissolved. The possible leakage of the heat-exchanging medium during the recovery of the blood can be precluded by clamping the flexible connection tube. The heat exchanger is very safe and easy to handle.

## Claims

1. Heat exchanger (101) for blood, comprising :

   a substantially cylindrical housing (109) having a heat-exchanging medium inlet tube (113) disposed at one end portion thereof, a heat-exchanging medium outlet tube (112) disposed at another end portion thereof, a blood inlet tube (105) and a blood outlet tube (106) disposed at circumferential portions between said medium inlet tube and said medium outlet tube and extending in directions substantially perpendicular to the longitudinal axis of said housing;

   a multiplicity of heat-exchanging cylindrical tubes (104) distributed cross-sectionally throughout the entire interior of the cylindrical housing and housed within said housing so as to extend in the longitudinal direction thereof, each of said heat-exchanging tube defining therein, an inner space (103) for passing the heat-exchanging medium; and

   a pair of partition walls (110a, 110b) secured to the inner wall of said housing at locations neighboring the opposite end parts thereof so as to form a pair of heat-exchanging medium chambers (111a, 111b) which respectively communicate with said medium inlet tube and said medium outlet tube, said partition walls respectively supporting thereon end portions of said heat-exchanging tubes to allow said heat-exchanging tubes to be separated from each other so as to define in said housing a blood chamber (102) which communicates with said blood inlet tube and said blood outlet tube, said partition walls allowing said inner spaces of the heat-exchanging tubes to communicate with said heat-exchanging medium chambers, characterized in that:

   said blood inlet tube (105) and said blood outlet tube (106) extend in directions substantially tangent to the outer circumferential surface of said housing (109) so that the blood in said blood chamber (102) comes into uniform contact with substantially all the heat exchanging tubes.

2. Heat exchanger as set forth in claim 1, wherein said said blood inlet port and said blood outlet port are disposed on opposite sides of said housing.

3. Heat exchanger as set forth in claim 2, wherein said blood inlet port and said blood outlet port extend in opposite directions.

4. Heat exchanger as set forth in claim 1, wherein said blood inlet port and said blood outlet port are offset from each other in the longitudinal direction of said housing.

23

**5.** Heat exchanger as set forth in claim 1, wherein said housing has a pair of blood passages (202a, 202b) which extend along the inner surface thereof between said partition walls and which respectively communicate with said blood inlet port and blood outlet port.

**6.** Heat exchanger as set forth in claim 5, wherein said blood passages have a substantially arch-shaped cross-section.

**7.** Heat exchanger as set forth in claim 1, wherein said housing has a rib (350) inwardly projecting on the inner surface thereof and which extends in the longitudinal direction thereof, said rib being disposed near said blood inlet port, so as to reflect a part of the blood, which is introduced from said blood inlet port into said blood chamber, toward the central axis of said housing.

**8.** Heat exchanger as set forth in claim 5, wherein said housing has a rib (350) inwardly projecting on the inner surface thereof and which extends in the longitudinal direction thereof, said rib being disposed near said blood inlet port, so as to reflect a part of the blood, which is introduced from said blood inlet port into said blood chamber, toward the central axis of said housing.

**9.** Heat exchanger as set forth in claim 7, wherein the distance between said rib and the adjacent heat-exchanging tube thereto is smaller than that between the adjacent heat-exchanging tubes.

**Patentansprüche**

**1.** Wärmeaustauscher (101) für Blut, umfassend:
- ein im wesentlichen zylindrisches Gehäuse (109), das ein an seinem einen Endabschnitt angeordnetes Wärmeaustauschmediumeinlaßrohr (113), ein an seinem anderen Endabschnitt angeordnetes Wärmeaustauschmediumauslaßrohr (112), ein Bluteinlaßrohr (105) und ein Blutauslaßrohr (106) aufweist, die an Umfangsabschnitten zwischen dem Mediumeinlaßrohr und dem Mediumauslaßrohr angeordnet sind und sich in Richtungen im wesentlichen senkrecht zur Längsachse des Gehäuses erstrecken;
- eine Vielzahl von zylindrischen Wärmeaustauschrohren (104), die im Querschnitt überall im gesamten Innenraum des zylindrischen Gehäuses verteilt sind und in dem Gehäuse so untergebracht sind, daß sie sich in dessen Längsrichtung erstrecken, wobei jedes der Wärmeaustauschrohre darin einen Innenraum (103) zum Durchlassen des Wärmeaustauschmediums begrenzt; und
- ein Paar von Trennwänden (110a, 110b), die an der Innenwand des Gehäuses an Stellen benachbart dessen gegenüberliegenden Endteilen befestigt sind, um ein Paar von Wärmeaustauschmediumkammern (111a, 111b) zu bilden, die jeweils mit dem Mediumeinlaßrohr und dem Mediumauslaßrohr in Verbindung stehen, wobei die Trennwände jeweils darauf Endabschnitte der Wärmeaustauschrohre tragen, um zu gestatten, daß die Wärmeaustauschrohre voneinander getrennt sind, um in dem Gehäuse eine Blutkammer (102) abzugrenzen, die mit dem Bluteinlaßrohr und dem Blutauslaßrohr in Verbindung steht, wobei die Trennwände es gestatten, daß die Innenräume der Wärmeaustauschrohre mit den Wärmeaustauschmediumkammern in Verbindung stehen,
dadurch **gekennzeichnet,** daß:
- sich das Bluteinlaßrohr (105) und das Blutauslaßrohr (106) in Richtungen im wesentlichen tangential zur Außenumfangfläche des Gehäuses (109) erstrecken, so daß das Blut in der Blutkammer (102) in gleichmäßigem Kontakt mit im wesentlichen sämtlichen Wärmeaustauschrohren gelangt.

**2.** Wärmeaustauscher nach Anspruch 1, bei dem die Bluteinlaßöffnung und die Blutauslaßöffnung auf gegenüberliegenden Seiten des Gehäuses angeordnet sind.

**3.** Wärmeaustauscher nach Anspruch 2, bei dem sich die Bluteinlaßöffnung und die Blutauslaßöffnung in entgegengesetzte Richtungen erstrecken.

**4.** Wärmeaustauscher nach Anspruch 1, bei dem die Bluteinlaßöffnung und die Blutauslaßöffnung voneinander in der Längsrichtung des Gehäuses versetzt sind.

**5.** Wärmeaustauscher nach Anspruch 1, bei dem das Gehäuse ein Paar von Blutkanälen (202a, 202b) aufweist, die sich entlang seiner Innenfläche zwischen den Trennwänden erstrecken und die jeweils mit der Bluteinlaßöffnung und der Blutauslaßöffnung in Verbindung stehen.

**6.** Wärmeaustauscher nach Anspruch 5, bei dem die Blutkanäle einen im wesentlichen bogenförmigen Querschnitt aufweisen.

**7.** Wärmeaustauscher nach Anspruch 1, bei dem das Gehäuse eine Rippe (350) aufweist, die auf seiner Innenfläche nach innen vorsteht und die sich in seiner Längsrichtung erstreckt, wobei die Rippe in der Nähe der Bluteinlaßöffnung angeordnet ist, um einen Teil des Blutes zur zentralen Achse des Gehäuses hin zu reflektieren, der von der Bluteinlaßöffnung in die Blutkammer eingeführt wird.

**8.** Wärmeaustauscher nach Anspruch 5, bei dem das Gehäuse eine Rippe (350) aufweist, die auf seiner Innenfläche nach innen vorsteht und die sich in seiner Längsrichtung erstreckt, wobei die Rippe in der Nähe der Bluteinlaßöffnung angeordnet ist, um einen Teil des Blutes zur zentralen Achse des Gehäuses hin zu reflektieren, der von der Bluteinlaßöffnung in die Blutkammer eingeführt wird.

**9.** Wärmeaustauscher nach Anspruch 7, bei dem der Abstand zwischen der Rippe und dem dieser benachbarten Wärmeaustauschrohr kleiner als derjenige zwischen den benachbarten Wärmeaustauschrohren ist.

**Revendications**

**1.** Echangeur de chaleur (101) pour du sang, comprenant:
une enveloppe sensiblement cylindrique (109) comportant un tube (113) d'entrée de fluide d'échange de chaleur disposé à une de ses parties d'extrémité, un tube (112) de sortie de fluide d'échange de chaleur disposé à son autre partie d'extrémité, un tube (105) d'entrée de sang et un tube (106) de sortie de sang disposé dans des parties circonférentielles entre ledit tube d'entrée de fluide et ledit tube de sortie de fluide et s'étendant dans des directions sensiblement perpendiculaires à l'axe longitudinal de ladite enveloppe;
une multiplicité de tubes cylindriques (104) d'échange de chaleur répartis sur la totalité de la section droite de l'intérieur complet de l'enveloppe cylindrique et logés à l'intérieur de ladite enveloppe de manière à s'étendre dans la direction longitudinale de cette dernière, chacun des tubes d'échange de chaleur définissant intérieurement un espace intérieur (103) pour le passage du fluide d'échange de chaleur; et
une paire de parois de séparation (110a, 110b) fixées à la paroi intérieure dudit boîtier à des endroits situés au voisinage des parties d'extrémité opposée de cette dernière de manière à former une paire de chambres (111a, 111b) de fluide d'échange de chaleur qui communiquent, respectivement, avec ledit tube d'entrée de fluide d'échange de chaleur et ledit tube de sortie de fluide d'échange de chaleur, lesdites parois de séparation supportant respectivement des parties d'extrémité desdits tubes d'échange de chaleur pour permettre auxdits tubes d'échange de chaleur d'être séparés les uns des autres de manière à définir dans ladite enveloppe une chambre (102) de sang qui communique avec ledit tube d'entrée de sang et ledit tube de sortie de sang, lesdites parois de séparation permettant auxdits espaces intérieurs des tubes d'échange de chaleur de communiquer avec lesdites chambres de fluide d'échange de chaleur, caractérisé en ce que:
le tube (105) d'entrée de sang et le tube (106) de sortie de sang s'étendent dans des directions sensiblement tangentes à la surface circonférentielle extérieure de l'enveloppe (109) de sorte que le sang se trouvant dans la chambre (102) de sang vient en contact uniforme avec sensiblement tous les tubes d'échange de chaleur.

**2.** Echangeur de chaleur selon la revendication 1, dans lequel ledit orifice d'entrée de sang et ledit orifice de sortie de sang sont disposés sur les côtés opposés de ladite enveloppe.

**3.** Echangeur de chaleur selon la revendication 2, dans lequel l'orifice d'entrée de sang et l'orifice de sortie de sang s étendent dans des directions opposées.

**4.** Echangeur de chaleur selon la revendication 1, dans lequel l'orifice d'entrée de sang et l'orifice de sortie de sang sont décalés l'un par rapport à l'autre dans la direction longitudinale de ladite enveloppe.

**5.** Echangeur de chaleur selon la revendication 1, dans lequel l'enveloppe comporte une paire de passages (202a, 202b) de sang qui s'étendent le long de la surface intérieure de l'enveloppe entre lesdites parois de séparation et qui communiquent respectivement avec ledit orifice d'entrée de sang et ledit orifice de sortie de sang.

**6.** Echangeur de chaleur selon la revendication 5, dans lequel les passages de sang ont sensiblement une section droite de forme arquée.

**7.** Echangeur de chaleur selon la revendication 1, dans lequel ladite enveloppe comporte une nervure (350) faisant saillie vers l'intérieur sur la surface intérieure de l'enveloppe et qui s'étend dans la direction longitudinale de cette dernière, ladite nervure étant disposée près de l'orifice d'entrée de sang de manière à renvoyer une partie du sang, qui est introduit par l'orifice d'entrée de sang, dans ladite chambre de sang, en direction de l'axe central de l'enveloppe.

**8.** Echangeur de chaleur selon la revendication 5, dans lequel ladite enveloppe comporte une nervure (350) faisant saillie vers l'intérieur sur la surface intérieure de l'enveloppe et qui s'étend dans la direction longitudinale de cette dernière, ladite nervure étant disposée près de l'orifice d'entrée de sang de manière à renvoyer une partie du sang, qui est introduit par ledit orifice d'entrée de sang, dans la chambre de sang, vers l'axe central de ladite enveloppe.

**9.** Echangeur de chaleur selon la revendication 7, dans lequel la distance entre ladite nervure et le tube d'échange de chaleur adjacent à cette dernière est plus petite que celle entre les tubes d'échange de chaleur adjacents.

26

FIG.1

FIG.2

# FIG.3

# FIG.4

# FIG.5

# FIG.6

FIG.7

# FIG.8

# FIG.9

EP 0 297 970 B1

# FIG.10

# FIG.11A

32

EP 0 297 970 B1

# FIG.11 B

302b

306

307

303

305

302a   305

A

# FIG.12

302b

303

307

305

350b

350a   302a

33

# FIG.13

402b

7

406

407

303

303

450

450

405

402a

# FIG.18

612

614

613

601

615

603

606

602

604

611b

609

# FIG.14

# FIG.15

FIG.17

103
157
108a
166
109
161

FIG.16

161
165
166
157
163
105
162
103
160
158
159

112
109
101
10
113